# EUROPEAN PATENT APPLICATION

(11) **EP 2 202 237 A1**
(43) Date of publication of application: **30.06.2010**
(21) Application number: 08306007.9
(22) Date of filing: 23.12.2008
(51) Int. Cl.: C07H 15/203, C07H 15/26, A61K 8/00, A61K 31/7034, A61P 35/00

(54) **Hydrosoluble [6)-O-alpha-d-glcp-(1->]n-6-o-bêta-d-glcp-(1->-phenllic derivatives with dermocosmetic, nutritional and therapeutic applications, and compositions containing said water soluble compounds**

(71) Applicant: Libragen, 31000 Toulouse (FR)
(72) Inventor: Auriol, Daniel, 31300, TOULOUSE (FR); Ginolhac, Aurélien, 31000, TOULOUSE (FR); Lefévre, Fabrice, 31190, AUTERIVE (FR); Nalin, Renaud, 31280, DREMIL-LAFAGE (FR)
(74) Representative: Gallois, Valérie

(57) **Abstract**

The invention relates to [6)-O-α-D-Glcp-(1→]ₙ-6-O-β-D-Glcp-(1→-phenolic derivatives. These [6)-O-α-D-Glcp-(1→]ₙ-6-O-β-D-Glcp-(1→phenolic derivatives of selected phenolics are new, have a solubility in water higher than that of their parent O-β-D-Glcp-(1→-phenolic derivatives and have useful applications in cosmetic, nutrition and pharmaceutical compositions, such as treating or preventing oxidative stress, a cancer, a cardiovascular disease, a bacterial infection, a viral infection, a fungal infection, a UV-induced erythema, an allergy, a metabolism disorder, diabetes, an obesity, an hormonal disorder, a bone disease, a pain, a brain disease, a mouth or teeth disease, an inflammatory or immune disorder.

## Description

### FIELD OF THE INVENTION

The present invention relates to water soluble [6)-O-α-D-Glcp-(1→]ₙ-6-O-β-D-Glcp-(1→-phenolic derivatives, compositions comprising such novel [6)-O-α-D-Glcp-(1→]ₙ-6-O-β-D-Glcp-(1→-phenolic derivatives, and their use for the beauty of the skin, for nutrition and for treating diseases.

### BACKGROUND OF THE INVENTION

### Phenolic compounds and their properties

Phenolic compounds (also called phenolics), or polyphenols, constitute one of the most numerous and widely-distributed groups of substances in the plant kingdom, with more than 8,000 phenolic structures currently known. Polyphenols are products of the secondary metabolism of plants. The expression "phenolic compounds" embraces a considerable range of substances that possess an aromatic ring bearing one or more hydroxyl substituents. Most of the major classes of plant polyphenol are listed in Table 1, according to the number of carbon atoms of the basic skeleton. The structure of natural polyphenols varies from simple molecules, such as phenolic acids, to highly polymerized compounds, such as condensed tannins (HARBORNE JB (1980 ) Plant phenolics. In: BELL EA, CHARLWOOD BV (eds) Encyclopedia of Plant Physiology, volume 8 Secondary Plant Products, Springer-Verlag, Berlin Heidelberg New York. Pp: 329-395). The three important groups for humans are phenolic acids (C6-C1, C6-C2 and C6-C3), flavonoids (C6-C3-C6) and high-molecular weight polyphenols (more than 30 carbon atoms). Indeed, the phenolics, particularly polyphenols, exhibit a wide variety of beneficial biological activities in mammals, including antiviral, antibacterial, glucose regulating, immune-stimulating, antiallergic, antihypertensive, antiischemic, antiarrhytmic, antithrombotic, hypocholesterolemic, antilipoperoxidant, hepatoprotective, anti-inflammatory, anticarcinogenic antimutagenic, antineoplastic, anti-thrombotic, and vasodilatory actions. They are powerful antioxidants *in vitro.*

**TABLE I : The major classes of phenolic compounds (or phenolics) in plants (HARBORNE JB, 1980)**

| NUMBER OF CARBON ATOMS | BASIC SKELETON | CLASS | EXAMPLES |
|---|---|---|---|
| 6 | C6 | Simple phenols Benzoquinones | Catechol, hydroquinone 2,6-Dimethoxybenzoquinone |
| 7 | C6-C1 | Phenolic acids | Gallic, salicylic |
| 8 | C6-C2 | Acetophenones Tyrosine derivatives Phenylacetic acids | 3-Acetyl-6-methoxybenzaldehyde Tyrosol p-Hydroxyphenylacetic |
| 9 | C6-C3 | Hydroxycinnamic acids Phenylpropenes Coumarins Isocoumarins Chromones | Caffeic, ferulic Myristicin, eugenol Umbelliferone, aesculetin Bergenon Eugenin |
| 10 | C6-C4 | Naphthoquinones | Juglone, plumbagin |
| 13 | C6-C1-C6 | Xanthones | Mangiferin |
| 14 | C6-C2-C6 | Stilbenes Anthraquinones | Resveratrol Emodin |
| 15 | C6-C3-C6 | Flavonoids Isoflavonoids | Quercetin, cyanidin Genistein |
| 18 | (C6-C3)2 | Lignans Neolignans | Pinoresinol Eusiderin |
| 30 | (C6-C3-C6)2 | Biflavonoids | Amentoflavone |
| n | (C6-C3)n (C6)n (C6-C3-C6)n | Lignins Catechol melanins Flavolans (Condensed Tannins) | |

Among the phenolic acids, the most important constitutive carbon frameworks are the hydroxybenzoic (C6-C1) and hydroxycinnamic (C6-C3) structures. The hydroxybenzoic acid content of edible plants is generally very low, with the exception of certain red fruits, black radish, and onions, which can have concentrations of several tens of milligrams per kilogram fresh weight. Hydroxybenzoic acids are components of complex structures such as hydrolyzable tannins (gallotannins in mangoes and ellagitannins in red fruits such as strawberries, raspberries and blackberries). The hydroxycinnamic acids are more common than are the hydroxybenzoic acids and consist chiefly of p-coumaric, caffeic, ferulic and sinapic acids. These acids are rarely found in the free form, except in processed food that has undergone freezing, sterilization or fermentation. The bound forms are glycosylated derivatives or esters of quinic acid, shikimic acid and tartaric acid. Caffeic acid and quinic acid combine to form chlorogenic acid, which is found in many types of fruit and in high concentration in coffee. Caffeic acid, both free and esterified, is generally the most abundant phenolic acid and represents between 75% and 100% of the total hydroxycinnamic acid of most fruit (MANACH C, SCALBERT A, MORAND C, REMESY C, JIMENEZ L (2004) Polyphenols: food sources and bioavailability. Am J Clin Nutr 79: 727-747).

The flavonoids consist of a large group of low-molecular weight polyphenolic substances, benzo-γ-pyrone derivatives that are diverse in chemical structure; they represent the most common and widely distributed group of plant phenolics. The flavonoids common structure is that of diphenylpropanes (C6-C3-C6); its consists of two aromatic rings (cycles A and B) linked through three carbons that usually form an oxygenated heterocycle (cycle C). Structural variations within the rings subdivide the flavonoids into several families: flavonols, flavones, flavanols, isoflavones, antocyanidins and others. These flavonoids often occur as glycosides, glycosylation usually rendering the molecule more water-soluble and less reactive toward free radicals. The flavonoid variants are all related by a common biosynthetic pathway, incorporating precursors from both the shikimate and the acetate-malonate pathways (CROZIER A, BURNS J, AZIZ AA, STEWART AJ, RABIASZ HS, JENKINS GI, EDWARDS CA, LEAN MEJ (2000) Antioxidant flavonols from fruits, vegetables and beverages: measurements and bioavailability. Biol Res 33: 79-88). Further modifications occur at various stages, resulting in an alteration in the extent of hydroxylation, methylation, isoprenylation, dimerization and glycosylation (producing O- or C-glycosides). Phenolic compounds act as antioxidants with mechanisms involving both free radical scavenging and metal chelation. Indeed, excess levels of metal cations of iron, zinc and copper in the human body can promote the generation of free radicals and contribute to the oxidative damage of cell membranes and cellular DNA; by forming complexes with these reactive metal ions, they can reduce their absorption and reactivity. It has to be underlined that though most flavonoids chelate Fe²⁺, there are large differences in the chelating activity. In particular, the dihydroflavonol taxifolin chelates more efficiently Fe²⁺ than the corresponding flavonol quercetin (VAN ACKER SABE, VAN DEN BERG DJ, TROMP MNJL, GRIFFIOEN DHG, VAN BENNEKOM, VAN DER VIJGH WJF, BAST A (1996) Structural aspects of antioxidant activity of flavonoids. Free Radic Biol Med 20: 331-342). Flavonoids have ideal structural chemistry for free radical-scavenging activities (several studies have shown the flavonoids to act as scavengers of superoxide anions, singlet oxygen, hydroxyl radicals and lipid peroxyl radicals by rapid donation of a hydrogen atom). One important finding from the studies of the relationship between the structural characteristics of flavonoids and their antiradical activity is that a catechol moiety (3',4'-dihydroxyphenol) on ring B is required for good scavenging activity. Recently, this statement was confirmed with nevertheless a modulation: in a study about the relationship between the structural characteristics of 29 flavonoids and their antiradical activity, it was indeed observed that the catechol structure in the B ring is not always a *conditio sine qua non* in achieving high free radical scavenging activity and that highly active flavonoids possess a 3',4'-dihydroxy B ring and/or a 3-OH group (AMIC D, DAVIDOVIC-AMIC D, BESLO D, TRINAJSTIC N (2003) Structure-radical scavenging activity relationships of flavonoids. Croatica Chem Acta 76: 55-61). Flavonoids have been shown to be more effective antioxidants *in vitro* than vitamins E and C on a molar basis (RICE-EVANS CA, MILLER NJ, PAGANGA G (1997) Antioxidant properties of phenolic compounds. Trends in Plant Science 2: 152-159). There are also reports of flavonoids inhibiting the activity of enzymes such as oxygenases.

It must be underlined that the hydrophobicity of polyphenols is intermediate between that of vitamin C (highly hydrophilic) and that of vitamin E (highly hydrophobic); polyphenols are thus expected to act at water-lipid interfaces and may be involved in oxidation regeneration pathways with vitamin C and E.

### Phenolyl glycosides

As previously mentioned, polyphenol can be found in nature under glycosylated forms. The sugar most commonly involved in glycoside formation is glucose, although galactose, rhamnose, xylose and arabinose also occur, as well as disaccharides such as rutinose (6-O-α-L-rhamnosyl-D-glucose). The most frequent natural glycosyl derivatives of polyphenols are O-β-D-Glucopyranoside ones. Among these compounds, compounds mentioned in Table II are of particular interest for their diverse biological properties .

**TABLE II : Some biological properties of natural phenolyl-O-β-D-Glucopyranoside compounds**

| Name | Non exhaustive list of biological properties of the phenolic compounds | Chemical Family |
|---|---|---|
| 4-deoxyphloridzin (4-deoxyphloretin-2'-O-β-D-glucopyranoside) | Antioxidant | Flavonoids |
| Apigetrin or Cosmosiin (Apigenin-7-O-β-D-glucopyranoside) | Antioxidant Antimicrobial | Flavonoids |
| Daidzin (Daidzein-7-O-β-D-glucopyranoside) | Antiallergic and antithrombotic Osteonecrosis prevention | Flavonoids |
| Pyracanthoside (Eriodictyol-7-O-glucopyranoside) | Antioxidant | Flavonoids |
| Flavanomarein (Isookanin-7-O-β-D-glucopyranoside) | Antioxidant | Flavonoids |
| Genistin (Genistein-7-O-β-D-glucopyranoside) | UV protection Reduction of bone loss | Flavonoids |
| Gossypin (Gossypetin-8-O-β-D-glucopyranoside) | Analgesia Anti-inflammatory | Flavonoids |
| Populnin (Kaempferol-7-O-β-D-glucopyranoside) | Antioxidant | Flavonoids |
| Cynaroside (Luteolin-7-O-β-D-glucopyranoside) | Anti-bacterial Anti-proliferative | Flavonoids |
| March (Okanin-4'-O-β-D-glucopyranoside) | Antioxidant | Flavonoids |
| Maritimein (Maritimetin-7-O-P-D-glucopyranoside) | Antioxidant | Flavonoids |
| Prunin (Naringenin-7-O-β-D-glucopyranoside) | Antioxidant | Flavonoids |
| Ononin (Formononetin-7-O-β-D-glucopyranoside) | Cell protection | Flavonoids |
| Phloridzin (Phloretin-2'-O-β-D-glucopyranoside) | Antiproliferative Glucose regulation | Flavonoids |
| Saponarin (Isovitexin-7-O-β-D-glucopyranoside) | Antioxidant Antibacterial | Flavonoids |
| Sissotrin (Biochanin A-7-O-β-D-glucopyranoside) | Glucose regulation | Flavonoids |
| Spiraeoside (Quercetin-4'-O-β-D-glucopyranoside) | Antioxidant Antiproliferative | Flavonoids |
| Helicin (Salicylaldehyde-O-β-D-Glucopyranoside) | Found in *Salix* species together with other phenol glycosides (e.g. salicin, salicortin, 2'-acetylsalicortin, populin, tremaculin, salidroside, triandrin and picein) | Phenolic acid |
| Picein (para- Hydroxyacetophenone-O-β-D-glucopyranoside) | Antixidant found in *Salvia offininalis* and *Rhodiola rosea* | Phenolic acid |
| Sennosides A and B | Laxative Alpha-glucoamylase inhibition | Quinone anthraquinone xanhone |
| Deoxyrhapontin (3,5-Dihydroxy-4'-methoxystilbene-3-O-β-D-glucopyranoside) | Glucose regulation | Stilben |
| Piceid (Resveratrol-3-O-β-D-glucopyranoside) | Ischemia/reperfusion injury protection Antioxidant | Stilben |
| Rhapontin (4'-Methoxy-3,3',5-stilbenetriol-3-O-β-D-glucopyranoside) | Lipid peroxidation inhibition Anti-proliferative Antithrombotic and antiallergic | Stilben |
| Trifolirhizin (Maackiain-3-O-β-D-glucopyranoside) | Skin whitening Anti-proliferative | (None) |
| Esculin (Esculetin-6-O-β-D-glucopyranoside) | Carcinogenesis prevention | Coumarins |
| Fraxin (Fraxetin-8-O-β-D-glucopyranoside) | Antioxidant | Coumarins |

Due to their low aqueous solubility, the use of phenolics in pharmaceutical, nutritional or cosmetic preparations requires adapted and specific formulations. Since these formulations must also satisfy the constraints associated with their final usage, the compromise between acceptability and aqueous concentration is often difficult to reach. Even when bearing a sugar moiety, many phenolics have a poor water solubility (most usually below 5 g/L at room temperature and at pH 7). Prior art information shows that despite the interest and abundance of phenolics, there are few reports on new phenolics compound derivatives being soluble in water. Some new water soluble polyphenol-O-α-D-Glucose derivatives obtained by an enzymatic process have been described in PCT patent application PCT/EP07/055815. Recently, Moon et al (2007, Appl Microbiol Biotechnol., Enzymatic synthesis and characterization of arbutin glucosides using glucansucrase from *Leuconostoc mesenteroides* B-1299CB) have shown the synthesis of arbutin-isomaltoside.

There is no description of phenolic compounds bearing a [6)-O-α-D-Glcp-(1→]ₙ-6-O-β-D-Glcp-(1→-moiety with n being equal or greater than 1, said glycoside polyphenolic compounds being at least 50% more soluble in water than the corresponding [6)-O-α-D-Glcp-(1→]ₙ-6-O-β-D-Glcp-(1→-compound.

Such new water soluble phenolics derivatives would be of great interest to include them easily in a wide range of products for dermocosmetics, nutrition of healthcare. It would also be useful to obtain derivatives from these phenolics which can be converted during their final usage in a metabolizable initial phenolics structure.

### SUMMARY OF THE INVENTION

This objective can be achieved by means of the present invention. Indeed, the inventors surprisingly observed that phenolic compounds bearing a [6)-β-D-Glcp-(1→]ₙ-6-O-β-D-Glcp-(1→-moiety with n being equal or greater than 1 are at least 50% more soluble in water the O-β-D-Glcp-(1→-phenolic compounds. Up to now, such phenolic compounds bearing a [6)-α-D-Glcp-(1→]ₙ-6-O-β-D-Glcp-(1→-moiety have never been disclosed.

Accordingly, the present invention relates to water soluble phenol derivatives having the following formula (I) : 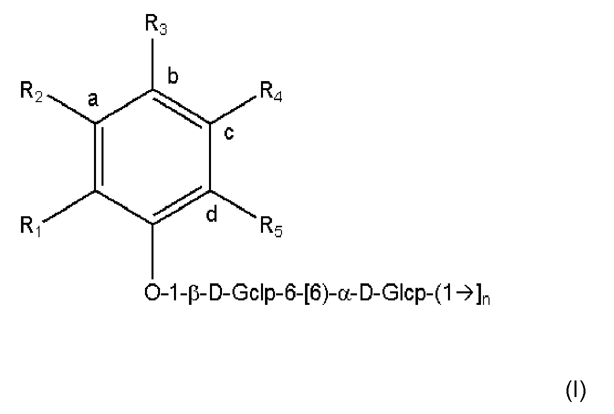 wherein
n is equal or greater than 1; more preferably n=1, 2, 3 or 4 and much more preferably n=1,
- R1: is selected from the group consisting of H, OH and OCH₃, preferably H or OH,
- R2: is selected from the group consisting of H, OH, OCH₃, and 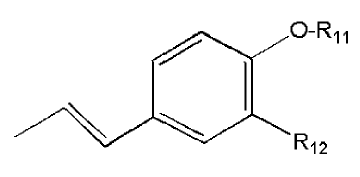
wherein R11 is H or CH₃ and R12 is H or OH,
and,
- R3: is selected from the group consisting of H, OH, COCH₃, 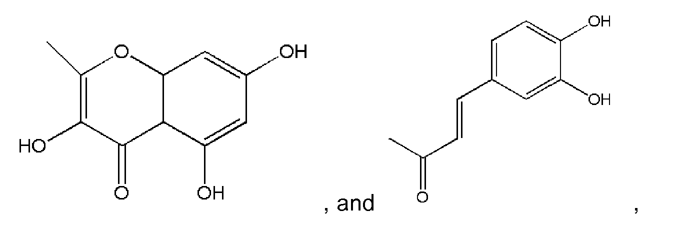
or,
R2 and R3 can together form an heteroring, when taken together with the atoms to which they are attached, said heterocyle being selected among the group consisting of: 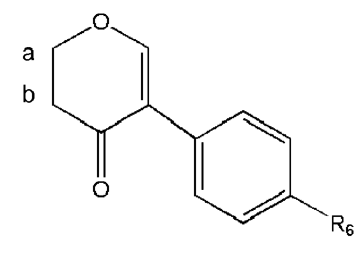 wherein R6 is H, OH or OCH₃, 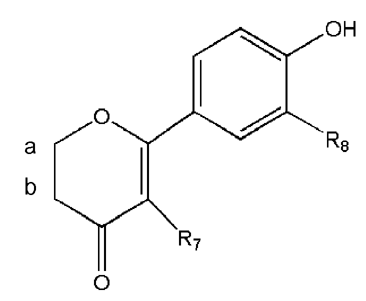 wherein R7 and R8 are selected from the group consisting of H, OH and OCH₃, preferably are H or OH, 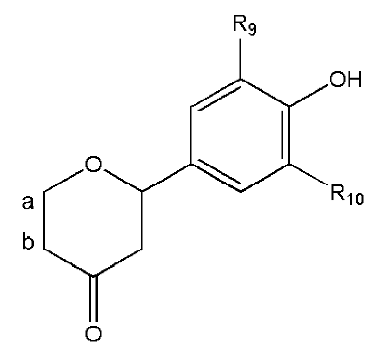 wherein R9 and R10 are selected from the group consisting of H, OH and OCH₃, preferably are H or OH, 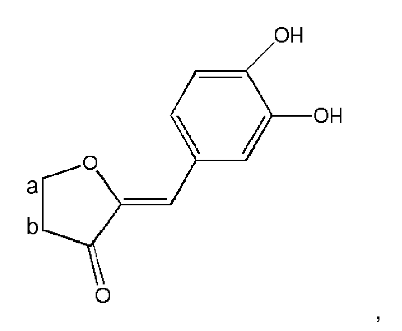 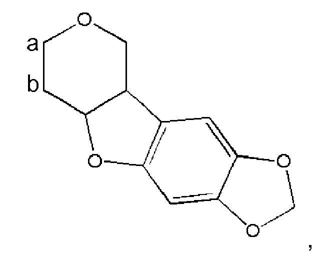 and 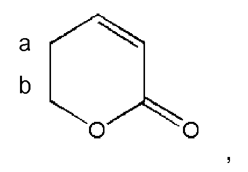 R4 is selected from the group consisting of H, OH and OCH₃, preferably is H or OH,
and
- R5: is selected from the group consisting of H, OH, 1-C-glucoside, COH, and 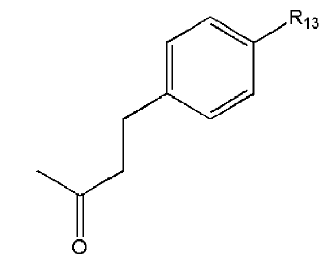
wherein R13 is selected from the group consisting of H, OH and OCH₃, preferably is H or OH,
or,
R4 and R5 can together form a ring, when taken with the atoms to which they are attached, said heterocyle being selected among the group consisting of: 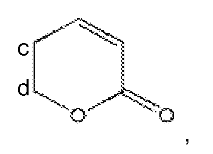 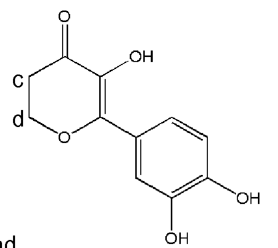 and 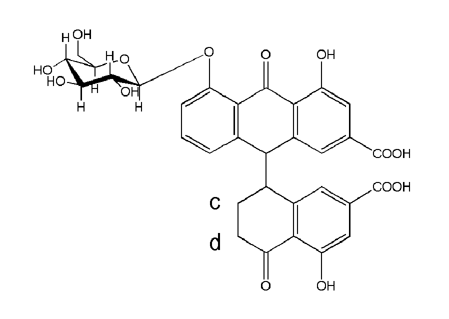 with the provisio that when R1, R2, R4, R5 are H then R3 is not OH,
or any salt thereof, in particular any pharmaceutically acceptable salt thereof.

In a preferred embodiment, the present invention relates to water soluble phenol derivatives having the following formula (I) : 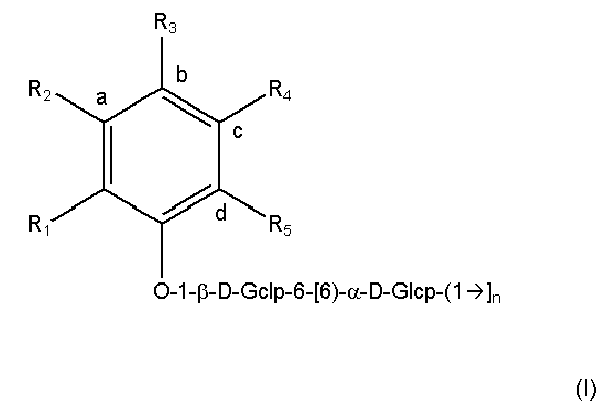 wherein
n is equal or greater than 1; more preferably n=1, 2, 3 or 4 and much more preferably n=1,
and R1, R2, R3, R4, R5 are selected among the following combinations :
a) R1 and R5 are H; R4 is H or OH; R2 and R3 together form, when taken together with the atoms to which they are attached, form a 6-membered heteroring, substituted by a phenol or phenoxy, of formula : 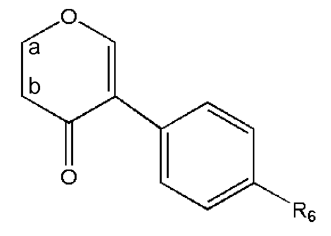 wherein R6 is OH or OCH₃; or
b) R1 is H, R4 is H or OH, R5 is H or 1-C-glucopyranoside, preferably at least one and only one among R4 and R5 being H, R2 and R3 together form when taken together with the atoms to which they are attached, a 6-membered heteroring, substituted by a phenol, of formula : 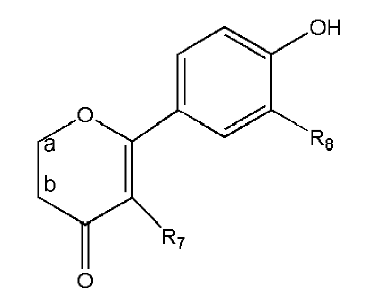 wherein R7 and R8 are H or OH, preferably R7 and R8 being not both OH, and more preferably when R7 or R8 represents OH, then R4 and R5 represent OH and H, respectively; or
c) R5 is H, R1 and R4 are H or OH, preferably at least one and only one among R1 and R4 being H, R2 and R3 together form, when taken together with the atoms to which they are attached, a 6-membered heteroring, substituted by a phenol, of formula : 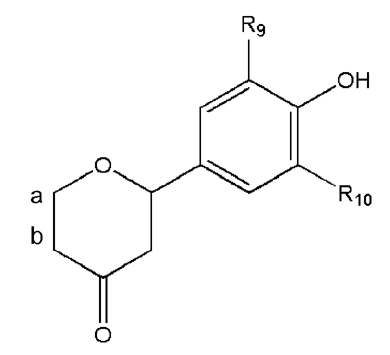 wherein R9 and R10 are H or OH, preferably R9 and R10 being not both OH; or
d) R1, R3 and R5 are H, R4 is OH,and R2 is 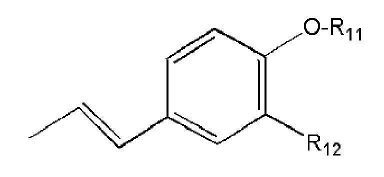 wherein R11 is H or CH₃ and R12 is H or OH; or
e) R1 and R3 are H, R2 and R4 are OH, and R5 is 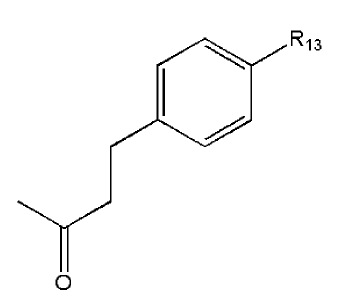 wherein R13 is H or OH, or
f) R1 and R2 are H, R5 is OH, R4 is H or OH and R3 is selected from the group consisting of 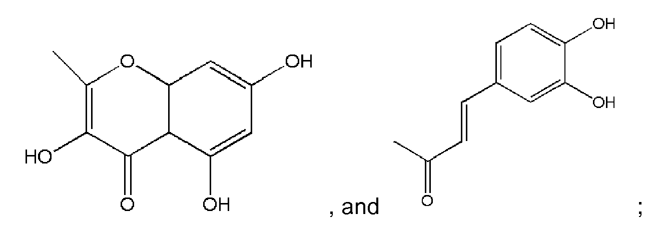 or
g) R1, R2 and R4 are H, R3 and R5 are either respectively COCH₃ and H, or H and COH; or
h) R1 is H or OH, R4 and R5 are H, R2 and R3 together form, when taken together with the atoms to which they are attached, a heteroring selected from the group consisting of: 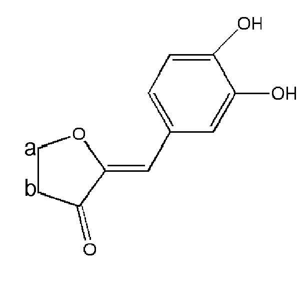 and 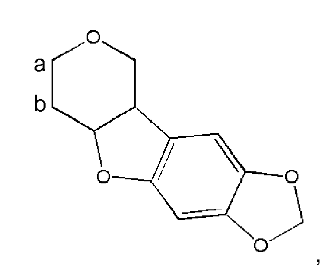 and preferably, when said heteroring is a 6-member ring, then R1 is H, and when said heteroring is a 5-member ring, then R1 is OH; or
i) R5 is OH, R1 and R4 are H, R2 and R3 together form, when taken together with the atoms to which they are attached, a 6-member heteroring of formula : 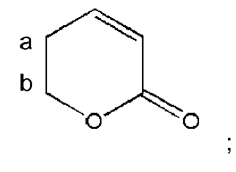 or
j) R1 and R3 are H or OH, R2 is H or OCH₃, R4 and R5 together form, when taken together with the atoms to which they are attached, a 6-member ring of formula selected in the group consisting of: 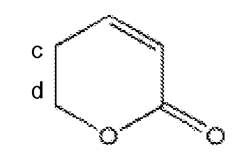 preferably with R1, R2 and R3 being OH, OCH₃ and H, respectively, 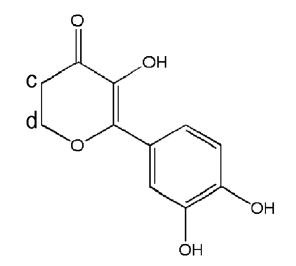 preferably with R1 and R3 being OH, and R2 being H, and 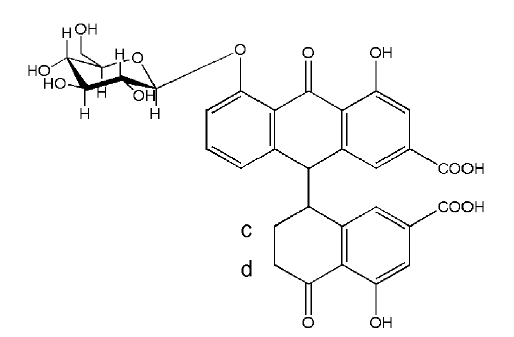 preferably with R1, R2 and R3 being all H.

A preferred [6)-O-α-D-Glcp-(1→]ₙ-6-O-β-D-Glcp-(1→-phenolic derivative is selected in the group consisting of [6)-O-α-D-Glcp-(1→]ₙ-6-O⁷-β-O-Glcp-(1→-daidzein (also named [6)-O-α-D-Glcp-(1→]ₙ-daidzin), [6)-O-α-D-Glcp-(1→]n-6-O⁷-β-D-Glcp-(1→-genistein (also named [6)-O-α-D-Glcp-(1→]ₙ-genistin), [6)-O-α-D-Glcp-(1→]ₙ-6-O⁷-β-D-Glcp-(1→-formononetin (also named [6)-α-D-Glcp-(1→]ₙ-ononin), [6)-O-α-D-Glcp-(1→]ₙ-6-O⁷-β-D-Glcp-(1→-biochanin A (also named [6)-O-α-D-Glcp-(1→]ₙ-sissotrin), [6)-O-α-D-Glcp-(1→]ₙ-6-O⁷-β-O-Glcp-(1→-apigenin (also named [6)-O-α-D-Glcp-(1→]ₙ-apigetrin), [6)-O-α-D-Glcp-(1→]ₙ-6-O⁷-β-O-Glcp-(1→-isovitexin (also named [6)-O-α-D-Glcp-(1→]ₙ-saponarin), [6)-O-α-D-Glcp-(1→]ₙ-6-O⁷-β-D-Glcp-(1→-luteolin (also named [6)-O-α-D-Glcp-(1→]ₙ-cynaroside), [6)-O-α-D-Glcp-(1→]ₙ-6-O⁷-β-D-Glcp-(1→-kaempferol (also named [6)-O-D-Glcp-(1→4]ₙ-populnin), [6)-O-α-D-Glcp-(1→]ₙ-6-O⁷-β-D-Glcp-(1→-naringenin (also named [6)-O-α-D-Glcp-(1→]ₙ-prunin), [6)-O-α-D-Glcp-(1→]ₙ-6-O⁷-β-D-Glcp-(1→-eriodictyol (also named [6)-O-α-D-Glcp-(1→]ₙ-pyracanthoside), [6)-O-α-D-Glcp-(1→]ₙ-6-O⁷-β-O-Glcp-(1→-isookanin (also named [6)-O-α-D-Glcp-(1→]ₙ-flavanomarein), [6)-O-α-D-Glcp-(1→]ₙ-6-O³-β-D-Glcp-(1→-3,5-dihydroxy-4'-methoxystilbene (also named [6)-O-α-D-Glcp-(1→]ₙ-deoxyrhapontin), [6)-O-α-D-Glcp-(1→]n-6-O³-β-D-Glcp-(1→-resveratrol (also named [6)-O-α-D-Glcp-(1→]ₙ-piceid), [6)-O-α-D-Glcp-(1→]ₙ-6-O³-β-D-Glcp-(1→-4'-methoxy-3,3',5-stilbenetriol (also named [6)-O-α-D-Glcp-(1→]ₙ-rhapontin), [6)-O-α-D-Glcp-(1→]ₙ-6-O^{2'}-β-D-Glcp-(1→-2',4',6'-trihydroxydihydrochalcone (also named [6)-O-α-D-Glcp-(1→]ₙ-2',4',6'-trihydroxydihydrochalcone-2'-O-glucoside or [6)-O-α-D-Glcp-(1→]ₙ-4-deoxyphloridzin), [6)-O-α-D-Glcp-(1→]ₙ-6-O^{2'}-β-D-Glcp-(1→-phloretin (also named [6)-O-α-D-Glcp-(1→]ₙ-phloridzin), [6)-O-α-D-Glcp-(1→]ₙ-6-O^{2'}-β-D-Glcp-(1→-okanin (also named [6)-O-α-D-Glcp-(1→]ₙ-marein), [6)-O-α-D-Glcp-(1→]ₙ-6-O^{4'}-β-O-Glcp-(1→-quercetin (also named [6)-O-α-D-Glcp-(1→]ₙ-spiraeoside), [6)-O-α-D-Glcp-(1→]ₙ-6-O⁴-β-O-Glcp-(1→-4-hydroxyacetophenone (also named [6)-O-α-D-Glcp-(1→]ₙ-picein), [6)-O-α-D-Glcp-(1→]ₙ-6-O²-β-O-Glcp-(1→-salicylaldehyde (also named [6)-O-α-D-Glcp-(1→]ₙ-helicin), [6)-O-α-D-Glcp-(1→]ₙ-6-O⁶-β-O-Glcp-(1→-maritimetin (also named [6)-O-α-D-Glcp-(1→]ₙ-maritimein), [6)-O-α-D-Glcp-(1→]ₙ-6-O³-β-D-Glcp-(1→-maackiain (also named [6)-O-α-D-Glcp-(1→]ₙ-trifolirhizin), [6)-O-α-D-Glcp-(1→]ₙ-6-O⁶-β-D-Glcp-(1→-esculetin (also named [6)-O-α-D-Glcp-(1→]ₙ-esculin), [6)-O-α-D-Glcp-(1→]ₙ-6-O⁸-β-O-Glcp-(1→-fraxetin (also named [6)-O-α-D-Glcp-(1→]ₙ-fraxin), [6)-O-α-D-Glcp-(1→]ₙ-6-O⁸-β-D-Glcp-(1→-gossypetin (also named [6)-O-α-D-Glcp-(1→]ₙ-gossypin), [6)-O-α-D-Glcp-(1→]ₙ-sennoside A and [6)-O-α-D-Glcp-(1→]ₙ-sennoside B.

The present invention also relates to a mixture of at least two [6)-O-α-D-Glcp-(1→]ₙ-6-O-β-D-Glcp-(1→-phenolic derivatives of the present invention.

In a preferred embodiment of the present invention, the [6)-O-α-D-Glcp-(1→]ₙ-6-O-β-D-Glcp-(1→-phenolic derivative of formula (I) has n=1, n=2, n=3, or n=4, more preferably n=1. Alternatively, n is greater than 1

Preferably the [6)-O-α-D-Glcp-(1→]ₙ-6-O-β-D-Glcp-(1→-phenolic derivative of the present invention has a solubility in water (at a temperature ranging from 4 to 80°C, and more preferably ranging from 12 to 37°C; and/or at pH ranging from 1.0 to 7.5, and more preferably ranging from 3.5 to 5.5) which is at least from 50% to 5000% higher than the solubility of the corresponding O-β-D-Glcp-(1→-phenolic derivative in the same conditions, and more preferably 50%, 100%, 200%, 400%, 800%, 1000%, 2000%, 3000%, 4000% or 5000%. In a preferred embodiment, the solubility is assessed in water at a temperature ranging from 4 to 80°C, and more preferably ranging from 12 to 37°C; and/or at pH ranging from 1.0 to 7.5, and more preferably ranging from 3.5 to 5.5.

The [6)-O-α-D-Glcp-(1→]ₙ-6-O-β-D-Glcp-(1→-phenolic derivative of the present invention can be cleaved by enzymes to release either the corresponding aglycone, or the corresponding [6)-O-α-D-Glcp-(1→]ₙ₋ₓ-6-O-β-D-Glcp-(1→-phenolic derivative wherein x=1 to n. Said enzymes are glycosidases such as amylase, pullulanase, O-α-glucosidase or O-β-glucosidase. O-α-glucosidase will enable the release of carbohydrate molecules of formula [6)-O-α-D-Glcp-(1→]ₙ₋ₓ wherein x=1 to n and of a O-β-D-Glcp-(1→)-phenolic derivative. O-β-glucosidase will enable the release of the aglycone part.

Preferably, said enzyme is issued from human associated micro-organisms, in particular human micro-organisms associated to skin, mouth, intestinal tract, upper respiratory system or female genital tract, even more preferably skin associated micro-organisms.

The present invention further concerns [6)-O-α-D-Glcp-(1→]ₙ-6-O-β-D-Glcp-(1→-phenolic derivative of the present invention as medicament, a nutritional complement or a cosmetics.

The present invention also concerns a pharmaceutical, nutritional, dermatological or cosmetic composition comprising a [6)-O-α-D-Glcp-(1→]ₙ-6-O-β-D-Glcp-(1→-phenolic derivative of the present invention. In particular, the composition can further comprise a glycosidase or a micro-organism expressing said glycosidase activity.

The present invention also concerns a product containing a [6)-O-α-D-Glcp-(1→]ₙ-n-6-O-β-D-Glcp-(1→-phenolic derivative of the present invention and a glycosidase or a micro-organism expressing said glycosidase activity as a combined preparation for simultaneous, separate or sequential use.

The present invention also concerns the use of a [6)-O-α-D-Glcp-(1→]ₙ-6-O-β-D-Glcp-(1→-phenolic derivative of the present invention for preparing a pharmaceutical, nutritional or cosmetic composition to be administered topically, orally, rectally, nasally, or vaginally, in particular wherein enzymes issued from micro-organisms associated to skin, mouth, intestinal tract, upper respiratory system or female genital tract release the corresponding aglycone.

The present invention also concerns the use of a [6)-O-α-D-Glcp-(1→]ₙ-6-O-β-D-Glcp-(1→-phenolic derivative of the present invention, a composition or a product of the invention for preparing a pharmaceutical or dermatological composition for treating or preventing a cancer, a cardiovascular disease, a bacterial infection, a viral infection, a fungal infection, a UV-induced erythema, an allergy, a metabolism disorder, diabetes, an obesity, an hormonal disorder, a bone disease, a pain, a brain disease, a mouth or teeth disease, an inflammatory or immune disorder.

### BRIEF DESCRIPTION OF THE DRAWINGS

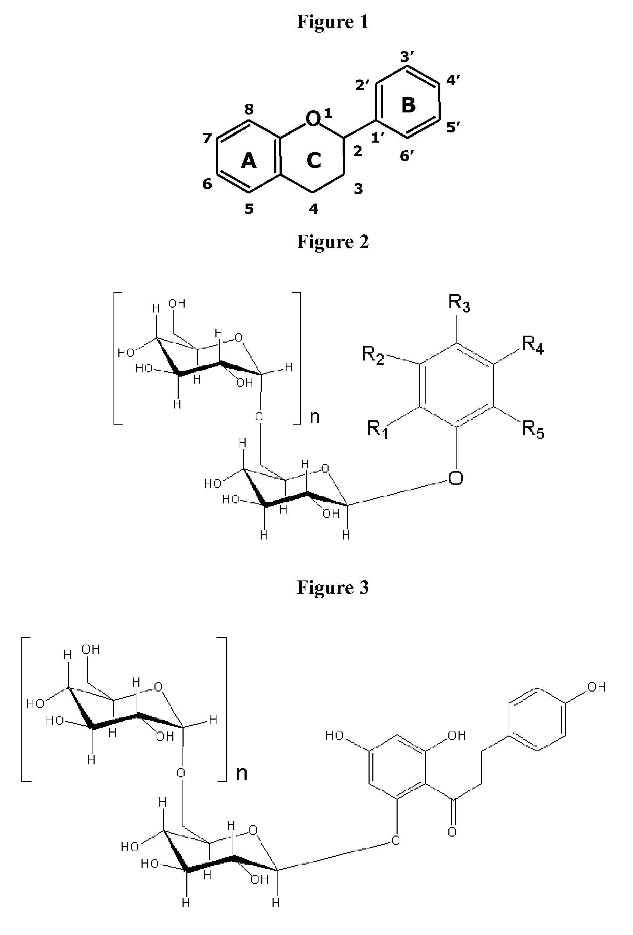

**Figure 1** - Flavonoids : basic structure and numbering of carbon atoms.
**Figure 2** - [6)-O-α-D-Glcp-(1→]ₙ-6-O¹-β-D-Glcp-(1→-phenol. The oxygen atom that is linked to the carbohydrate moiety of the molecule is supported by the carbon atom in the phenyl ring having the number 1. For this reason, the number "1" is mentioned in the form of an exponent just after the representation of the oxygen atom of the phenolic aglycone.
**Figure 3** - [6)-O-α-D-Glcp-(1→]ₙ-6-O^{2'}-β-D-Glcp-(1→-phloretin. The oxygen atom that is linked to the carbohydrate moiety of the molecule is supported by the carbon atom in the phenyl ring having the number 2' For this reason, the number "2' is mentioned in the form of an exponent just after the representation of the oxygen atom of the phenolic aglycone.

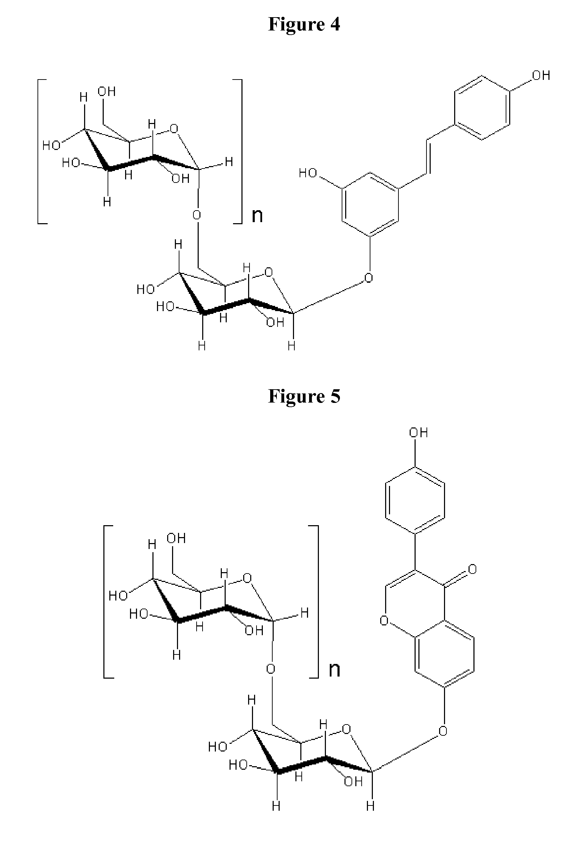

**Figure 4** - [6)-O-α-D-Glcp-(1→]ₙ-6-O³-β-O-Glcp-(1→-resveratrol. The oxygen atom that is linked to the carbohydrate moiety of the molecule is supported by the carbon atom in the phenyl ring having the number 3. For this reason, the number "3" is mentioned in the form of an exponent just after the representation of the oxygen atom of the phenolic aglycone.
**Figure 5** - [6)-O-α-D-Glcp-(1→]ₙ-6-O⁷-β-D-Glcp-(1→-daidzein. The oxygen atom that is linked to the carbohydrate moiety of the molecule is supported by the carbon atom in the flavonoids structure having the number 7 (see Fig 1). For this reason, the number "7" is mentioned in the form of an exponent just after the representation of the oxygen atom of the aglycone.

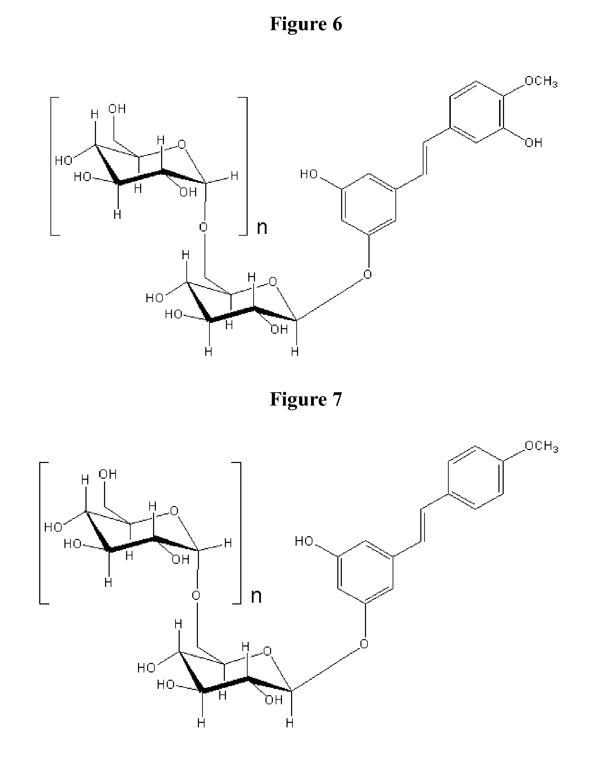

**Figure 6** - [6)-O-α-D-Glcp-(1→]ₙ-6-O³-β-O-Glcp-(1→-4'-methoxy-3,3',5-stilbenetriol. The oxygen atom that is linked to the carbohydrate moiety of the molecule is supported by the carbon atom in the phenyl ring having the number 3. For this reason, the number "3" is mentioned in the form of an exponent just after the representation of the oxygen atom of the phenolic aglycone.
**Figure 7** - [6)-O-α-D-Glcp-(1→]ₙ-6-O³-D-Glcp-(1→-3,5-dihydroxy-4'-methoxystilbene. The oxygen atom that is linked to the carbohydrate moiety of the molecule is supported by the carbon atom in the phenyl ring having the number 3. For this reason, the number "3" is mentioned in the form of an exponent just after the representation of the oxygen atom of the phenolic aglycone.

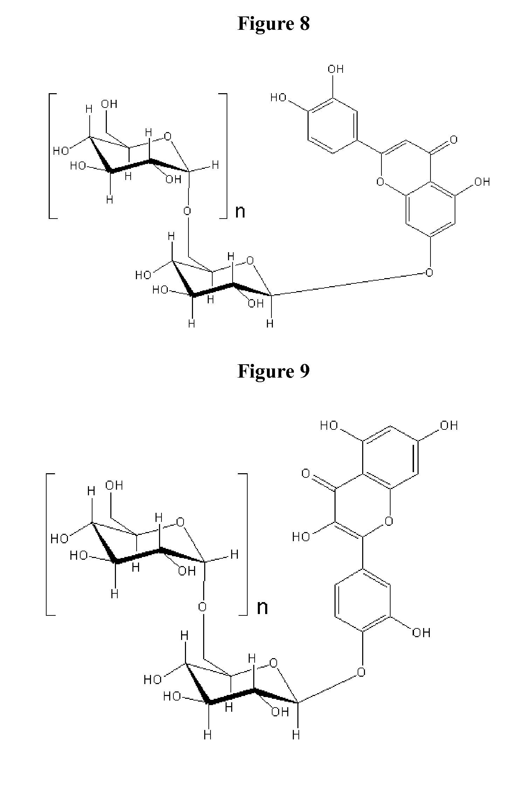

**Figure 8** - [6)-O-α-D-Glcp-(1→]ₙ-6-O⁷-β-D-Glcp-(1→-luteolin. The oxygen atom that is linked to the carbohydrate moiety of the molecule is supported by the carbon atom in the flavonoids structure having the number 7 (see Fig 1). For this reason, the number "7" is mentioned in the form of an exponent just after the representation of the oxygen atom of the aglycone.
**Figure 9** - [6)-O-α-D-Glcp-(1→]ₙ--6-O^{4'}-β-O-Glcp-(1→-quercetin. The oxygen atom that is linked to the carbohydrate moiety of the molecule is supported by the carbon atom in the flavonoids structure having the number 4' (see Fig 1). For this reason, the number "4"' is mentioned in the form of an exponent just after the representation of the oxygen atom of the aglycone.

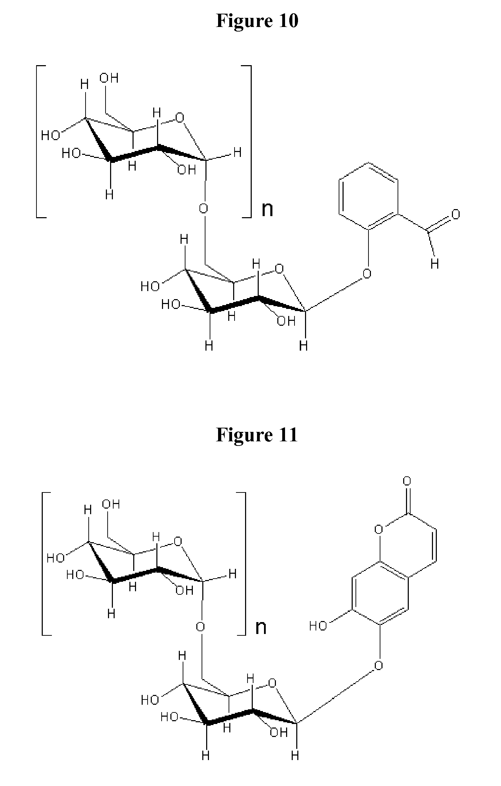

**Figure 10** - [6)-O-α-D-Glcp-(1→]ₙ-6-O²-β-O-Glcp-(1→-salicylaldehyde. The oxygen atom that is linked to the carbohydrate moiety of the molecule is supported by the carbon atom in the phenyl ring having the number 2. For this reason, the number "2" is mentioned in the form of an exponent just after the representation of the oxygen atom of the phenolic aglycone.
**Figure 11** - [6)-O-α-D-Glcp-(1→]ₙ-6-O⁶-β-O-Glcp-(1→-esculetin. The oxygen atom that is linked to the carbohydrate moiety of the molecule is supported by the carbon atom in the esculetin structure having the number 6. For this reason, the number "6" is mentioned in the form of an exponent just after the representation of the oxygen atom of the aglycone.

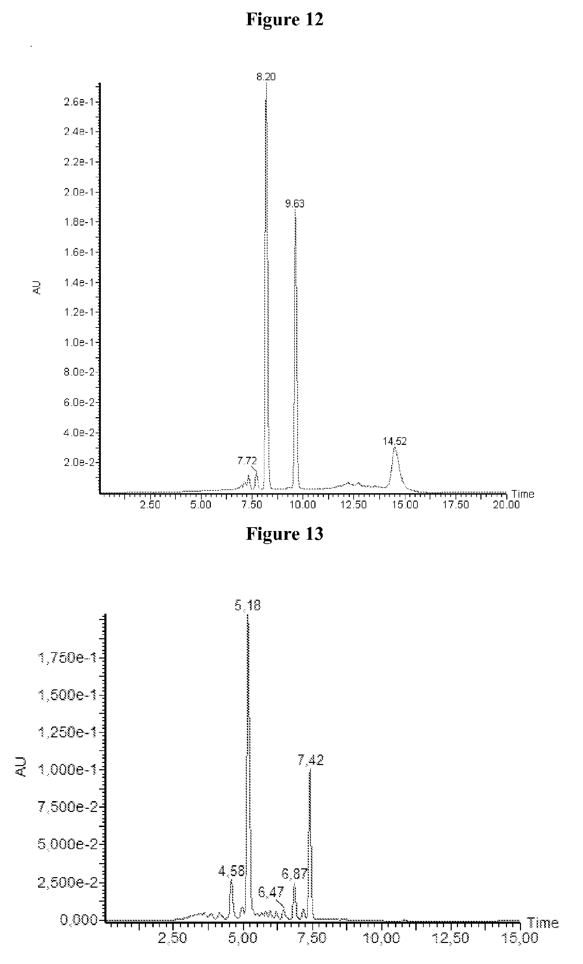

**Figure 12** - HPLC chromatogram of the reaction medium containing [6)-O-α-D-Glcp-(1→]ₙ-6-O^{2'}-D-Glcp-(1→-phloretin (285 nm). Retention time of O^{2'}-β-D-Glcp-(1→-phloretin (phloridizin): 9.63 min. Conditions for MeOH containing 1% acetic acid: 20 to 45% from 0 to 4 min - 45 to 55% from 4 to 8 min - 55 to 80% from 8 to 9 min - 80% from 9 to 11 min - 80 to 20% from 11 to 13 min.
**Figure 13** - HPLC chromatogram of the reaction medium containing [6)-O-α-D-Glcp-(1→]ₙ-6-O³-β-D-Glcp-(1→-4'-methoxy-3,3',5-stilbenetriol and [6)-O-α-D-Glcp-(1→]ₙ-6-O³-β-D-Glcp-(1→-3,5-dihydroxy-4'-methoxystilbene (320 nm). Retention time of O³-P-D-Glcp-(1→-4'-methoxy-3,3',5-stilbenetriol (rhapontin): 5.18 min. Retention time of O³-β-D-Glcp-(1→-3,5-dihydroxy-4'-methoxystilbene (deoxyrhapontin): 7.42 min. Conditions for MeOH containing 1% acetic acid: 40% from 0 to 1 min - 40 to 90% from 1 to 8 min - 90% from 8 to 11 min - 90 to 40% from 11 to 13 min.

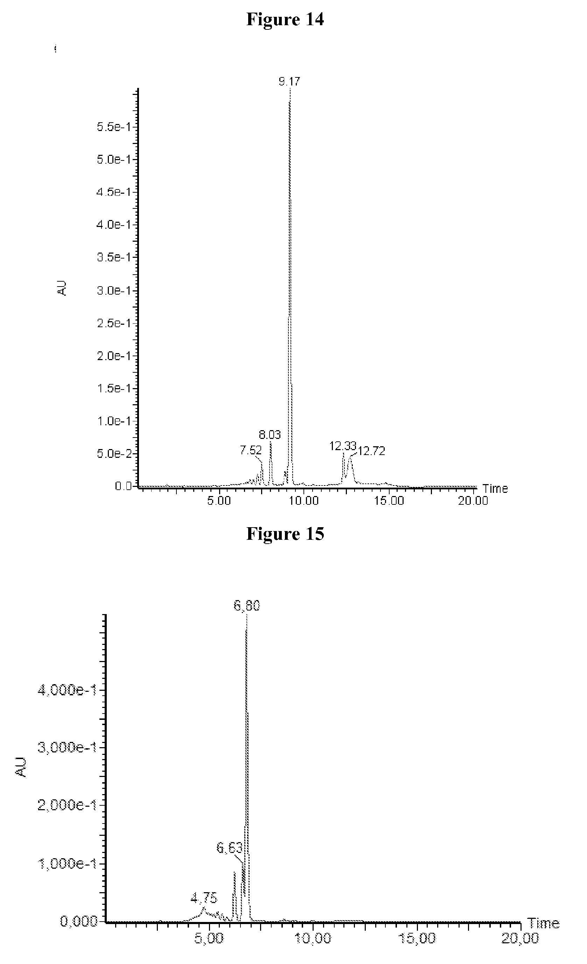

**Figure 14** - HPLC chromatogram of the reaction medium containing [6)-O-α-D-Glcp-(1→]ₙ-6-O⁷-β-D-Glcp-(1→-naringenin (284 nm). Retention time of O⁷-β-D-Glcp-(1→-naringenin (prunin): 5.18 min. Conditions for MeOH containing 1% acetic acid: 20 to 45% from 0 to 4 min - 45 to 55% from 4 to 8 min - 55 to 80% from 8 to 9 min - 80% from 9 to 11 min - 80 to 20% from 11 to 13 min.
**Figure 15** - HPLC chromatogram of the reaction medium containing [6)-O-α-D-Glcp-(1→]ₙ-6-O³-β-D-Glcp-(1→-resveratrol (318 nm). Retention time of O³-β-O-Glcp-(1→-resveratrol (piceid, polydatin): 6.80 min. Conditions for MeOH containing 1% acetic acid: 20 to 45% from 0 to 4 min - 45 to 55% from 4 to 8 min - 55 to 80% from 8 to 9 min - 80% from 9 to 11 min - 80 to 20% from 11 to 13 min.

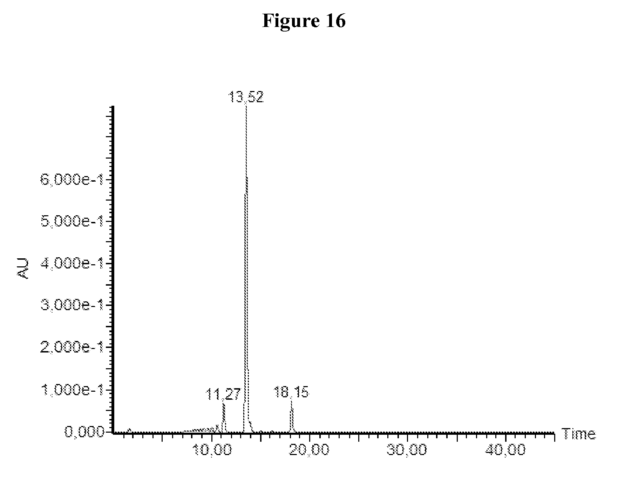

**Figure 16** - HPLC chromatogram of the reaction medium containing [6)-O-α-D-Glcp-(1→]ₙ-6-O⁶-β-D-Glcp-(1→-esculetin (335 nm). Retention time of [6)-O-α-D-Glcp-(1→]ₙ-6-O⁶-β-O-Glcp-(1→-esculetin (esculin): 13.52 min. Conditions for MeOH containing 1% acetic acid: 10 to 20% from 0 to 10 min - 20 to 50% from 10 to 25 min - 50% from 25 to 30 min - 50 to 10% from 30 to 35 min.

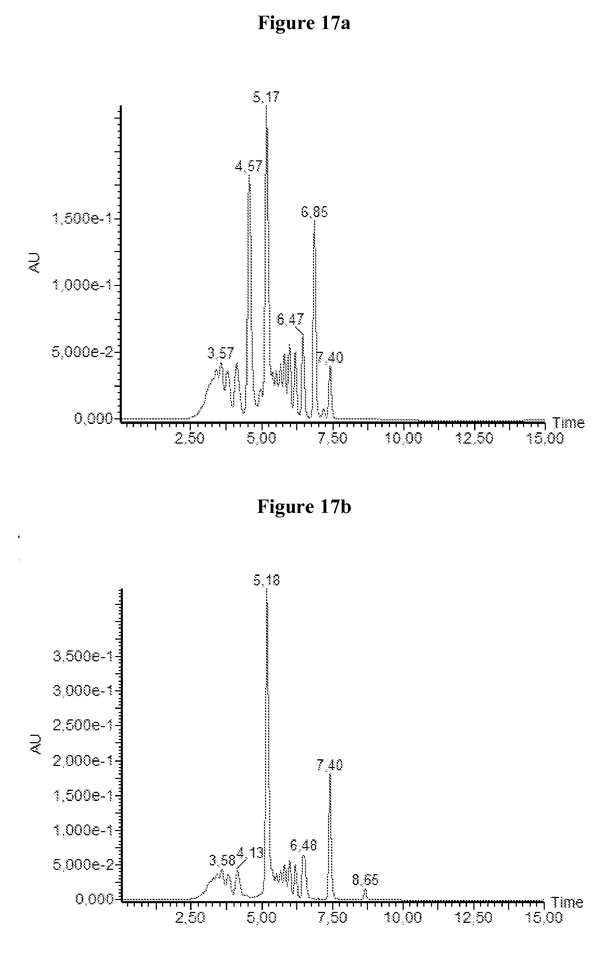

**Figure 17** - HPLC chromatogram of a reaction medium containg [6)-O-α-D-Glcp-(1→]ₙ-6-O³-β-D-Glcp-(1→-4'-methoxy-3,3',5-stilbenetriol and [6)-O-α-D-Glcp-(1→]ₙ-6-O³-β-D-Glcp-(1→-3,5-dihydroxy-4'-methoxystilbene Fig. 17/ before and Fig. 17/ after a treatment with an α-glucosidase (320 nm). Treatment with an α-glucosidase (FLUKA, 70797) at 1 mg/ml in a solution containing the glucosides at a concentration close to 1 mg/ml - pH 6.3 (PIPES, 25 mM) - 3 hours at 30°C. Conditions for MeOH containing 1% acetic acid: 40% from 0 to 1 min - 40 to 90% from 1 to 8 min - 90% from 8 to 11 min - 90 to 40% from 11 to 13 min.

Structures shown in figures 3-11 are given as illustrations. Other [6)-O-α-D-Glcp-(1→]ₙ-6-O-β-D-Glcp-(1→-phenolic derivative are also contemplated in the present invention.

### DETAILED DESCRIPTION OF THE INVENTION

### Definitions

**Phenolic compound or Phenolics or Phenolic derivative** : compound that possess an aromatic ring bearing one or more hydroxyl substituents.
**Flavonoids :** polyphenolic compounds possessing 15 carbon atoms, two benzene rings joined by a linear three carbon chain giving a system C6-C3-C6. The first benzene ring (ring A) forms with an oxygen atom and the three carbon atoms joining the two benzene rings a chromane skeleton (rings A and C). The chromane skeleton bears the second aromatic ring B in position 2, 3 or 4. In a few cases, the six-membered heterocyclic ring C occurs in an isomeric open form or is replaced by a five-membered ring.
**Enzyme :** protein molecule that catalyses chemical reactions on molecules (named substrates) to obtain other molecules (named products). A recommended name, a systematic name which stresses the type of reaction and an Enzyme Commission (EC) code number are assigned to each enzyme. These code numbers, prefixed by EC, contain four elements separated by points. The first number shows to which of the six main divisions (classes) the enzyme belongs: oxidoreductases (EC 1), transferases (EC 2), hydrolases (EC3), lyases (EC4), isomerases (EC5) and ligases (EC6). The second number indicates the subclass, the third the sub-subclass and the fourth is the serial number of the enzyme in its sub-subclass.
**Glycosidase :** Glycosidases are glycoside hydrolase enzymes categorized under the EC number 3.2
**Glucosidase :** Glucosidases are glycoside hydrolase enzymes categorized under the EC number 3.2.1. β-glucosidase is a glucosidase enzyme which acts upon β-glucosidic bonds linking two glucose or glucose-substituted molecules. α-glucosidase is a glucosidase enzyme which acts upon α-glucosidic bonds linking two glucose or glucose-substituted molecules.
**Bioavailability :** the degree to which or rate at which a molecule or other substance is absorbed or becomes available at the site of physiological activity after administration or application.
**Glycone :** chemical part of a glycosidic derivative which belongs to the carbohydrate family. If the glycone group is glucose, then the molecule is a glucoside; if it is fructose, then the molecule is a fructoside; if it is glucuronic acid, then the molecule is a glucuronide.
**Glcp :** refers to a glucopyranosyl group.
**Glycosidic bond :** chemical linkage between a glycone and an other glycone or a aglycone. Depending on whether the glycosidic bond lies "below" or "above" the plane of the cyclic carbohydrate molecule when considering the HAWORTH projection, glycosides are classified as α-glycosides or β-glycosides.
**Aglycone :** Chemical part of a glycosidic derivative which is not the glycone one.
Where "comprising" is used, this can preferably be replaced by "consisting essentially of", more preferably by "consisting of".
**"Heterocycle"** groups are groups containing 1 to 5 rings, fused or not fused, comprising one or more heteroatoms, preferably 1 to 5 endocyclic heteroatoms. They may be mono-, bi- or tri-cyclic. They may be aromatic or not. Examples of aromatic heterocycles include pyridine, pyridazine, pyrimidine, pyrazine, furan, thiophene, pyrrole, oxazole, thiazole, isothiazole, imidazole, pyrazole, oxadiazole, triazole, thiadiazole and triazine groups. Examples of bicycles include in particular quinoline, isoquinoline and quinazoline groups (for two 6-membered rings) and indole, benzimidazole, benzoxazole, benzothiazole and indazole (for a 6-membered ring and a 5-membered ring). Nonaromatic heterocycles comprise in particular piperazine, piperidine, etc.
**"Heteroatom"** denotes N, S, or O.

### Preparation of the [6)-O-α-D-Glcp-(1→]ₙ-6-O-β-D-Glcp-(1→-phenolic derivative

Both chemical and biochemical (enzymatic) approaches known from the man of the art can be used to prepare the [6)-O-α-D-Glcp-(1→]ₙ-6-O-β-D-Glcp-(1→-phenolic derivative of the present invention. Enzymatic reactions can either involve α-glucosidase (EC 3.2.1.20) glycosyltransferases (EC 2.4.1.5) as described in EP2007/055815. Man of the art will adapt said synthesis methods to the intrinsic properties (solubility, stability, ...) of the initial β-D-Glcp-(1→-phenolic derivative from which the [6)-O-α-D-Glcp-(1→]ₙ-6-O-β-D-Glcp-(1→-phenolic derivative is synthetized.

In one embodiment of the present invention, conditions allowing the analytical characterization of the synthesized derivatives can be as follows :
The synthesis media can be analyzed by high performance liquid chromatography coupled with a photodiode array detector (PDA Waters® 996) and a mass spectrometer (Micromass ZQ 2000, Waters®).
Operating conditions for chromatography:
   Column: KROMASIL C18 5µ, 250 mm x 4.6 mm (reference: K2185; A.I.T.; 117 rue de Stalingrad; 78800 Houilles)

### Elution (method N#1):

solvent A: deionized water containing 1% v/v acetic acid
solvent B: HPLC grade methanol containing 1% v/v acetic acid
0 to 4 minutes: 80% to 55% A (linear); 20% to 45% B (linear); 1 ml/minute
4 to 8 minutes: 55% to 45% A (linear); 45% to 55% B (linear); 1 ml/minute
8 to 9 minutes: 45% to 20% A (linear); 55% to 80% B (linear); 1 ml/minute
9 to 11 minutes: 20% A; 80% B; 1 ml/minute
11 to 13 minutes: 20% to 80% A (linear); 80% to 20% B (linear); 1 ml/minute
20 minutes: next injection

### Elution (method N#2):

solvent A: deionized water containing 1% v/v acetic acid
solvent B: HPLC grade methanol containing 1% v/v acetic acid
0 to 1 minutes: 60% A; 40% B; 1 ml/minute
1 to 8 minutes: 60% to 10% A (linear); 40% to 90% B (linear); 1 ml/minute
8 to 11 minutes: 10% A; 90% B; 1 ml/minute
11 to 13 minutes: 10% to 60% A (linear); 90% to 40% B (linear); 1 ml/minute
20 minutes: next injection

### Elution (method N#3):

solvent A: deionized water containing 1% v/v acetic acid
solvent B: HPLC grade methanol containing 1% v/v acetic acid
0 to 10 minute: 90% to 80% A (linear); 10% to 20% B; 1 ml/minute
10 to 25 minutes: 80% to 50% A (linear); 20% to 50% B (linear); 1 ml/minute
25 to 30 minutes: 50% A; 50% B; 1 ml/minute
30 to 35 minutes: 50% to 90% A (linear); 50% to 10% B (linear); 1 ml/minute
45 minutes: next injection

Colum temperature: 40°C
Injection volume: 10 µL

Photodiode array detector
Start wavelength: 210 nm
End wavelength: 400 nm
Resolution: 1.2 nm
Sampling rate: 1 spectra/second

LC mass spectrometer (single quadripole)
lonisation: electrospray in negative mode
Spray voltage: 3.0 kV
Source temperature: 150°C
Cone tension: 20 or 40 V
Extractor: 3.0 V
Desolvation temperature: 300°C
Cone gas flow: 30 L/hour
Desolvation gas flow: 600 L/hour
Full scan mass spectra: m/z from 100 to 2000

### Purification

After synthesis, [6)-O-α-D-Glcp-(1→]ₙ-6-O-β-D-Glcp-(1→-phenolic derivative can be either used directly or purified to reach a desired purity in terms of residual compounds, including sugars, optionnaly enzyme and co-solvents.

For example, the [6)-O-α-D-Glcp-(1→]ₙ-6-O-β-D-Glcp-(1→-phenolic derivative can be adsorbed on a synthetic macroporous adsorbent resin by taking advantage of the difference of absorbing ability of substances. Due to the presence of residual substances in the intersticial volume, the resin with the adsorbed phenolic substances is washed with water in order to completely flush out the residual compounds, the sugars and the polysaccharide and the co-solvent. Then the resin can undergo an elution step with an appropriate solvent to recover the synthesized product. The appropriate solvent can be for example pure methanol, ethanol, n-propanol, 2-propanol, acetone or a mixture of them or a mixture of them with water. The solution containing the synthesized product(s) can be concentrated by evaporation under vacuum at moderate temperature (not higher than 50°C) or with compatible membrane equipments for further purification, or directly used for further purification. Further purification steps such as liquid/liquid extraction, preparative HPLC, or other rounds of resin purification can be used to attain the required level of purity for the final application. Organic solvent that can be used for liquid-liquid extraction are ethyl acetate, butyl acetate, methyl ethyl ketone, depending on the solubility difference of the phenolic compound and phenolic compound glucoside.

Finally, a syrup containing the desired substance(s) can be obtained by removing the solvent (water or organic solvent) by evaporation under vacuum at moderate temperature (not higher than 50°C) or with compatible membrane equipments and concentrating the resulting solution to give a prescribed concentration. This syrup can be dried (freeze drying, spray drying or any other way of drying that will preserve the integrity of the molecules) to obtain a powder.

The synthetic macroporous adsorbent resin can be used either in a tank (a sieve with a convenient mesh depending on the resin granulometry will be used to recover the resin) or located in a column fed with a pump. By synthetic macroporous adsorbent resin, it is understood non ionic and porous synthetic resins which have relatively large surface area such as those containing styrene - divinyl benzene copolymer, phenolformaldehyde polymers, acrylic polymer and methacrylic polymer.

The present invention relates to water soluble phenol derivatives having the following formula (I) : 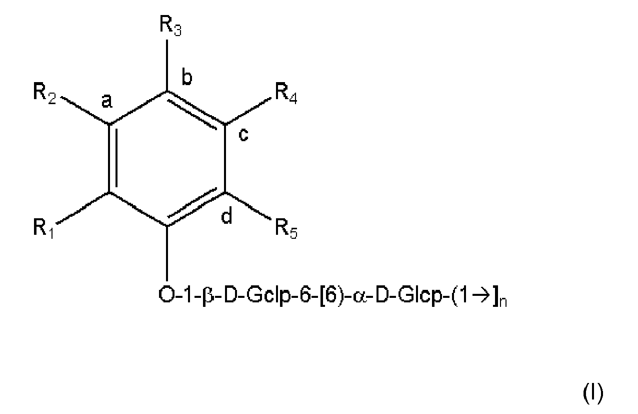 wherein
n is equal or greater than 1; more preferably n=1, 2, 3 or 4 and much more preferably n=1,
- R1: is selected from the group consisting of H, OH and OCH₃, preferably is H or OH,
- R2: is selected from the group consisting of H, OH, OCH₃, and 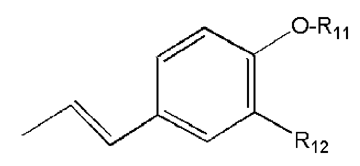
wherein R11 is H or CH₃ and R12 is H or OH,
and,
- R3: is selected from the group consisting of H, OH, COCH₃, 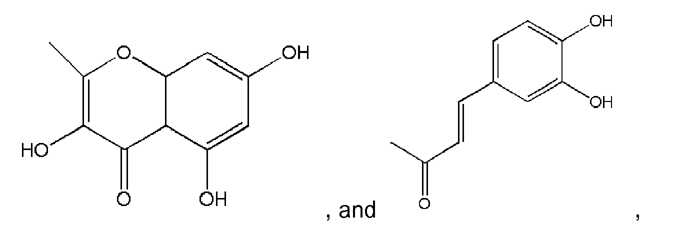
or,
R2 and R3 can together form an heteroring, when taken together with the atoms to which they are attached, said heterocyle being selected among the group consisting of : 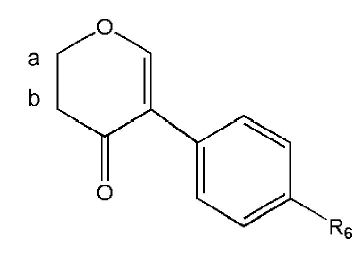 wherein R6 is H, OH or OCH₃, 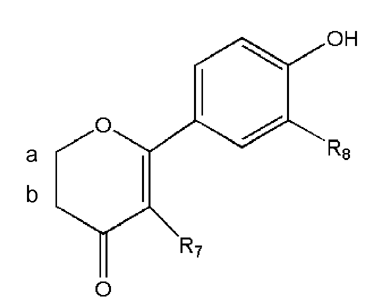 wherein R7 and R8 are selected from the group consisting of H, OH and OCH₃, preferably are H or OH, 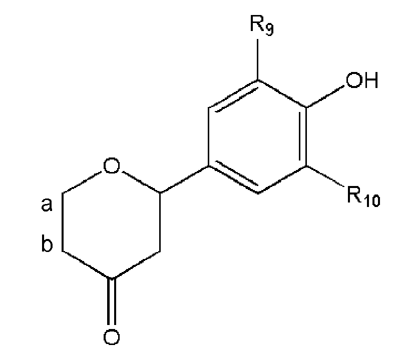 wherein R9 and R10 are selected from the group consisting of H, OH and OCH₃, preferably are H or OH, 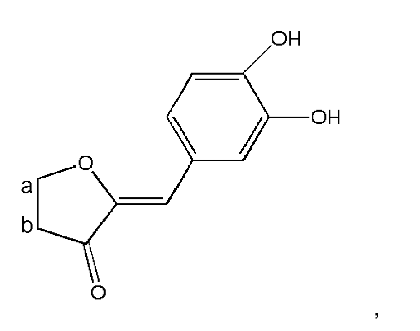 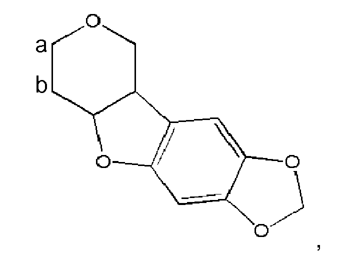 and 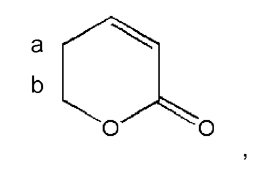
- R4: is selected from the group consisting of H, OH and OCH₃, preferably is H or OH,
and
- R5: is selected from the group consisting of H, OH, 1-C-glucoside, COH, 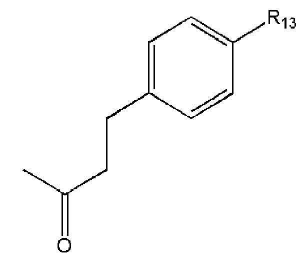
wherein R13 is selected from the group consisting of H, OH and OCH₃, preferably is H or OH,
or,
R4 and R5 can together form a ring, when taken with the atoms to which they are attached, said heterocyle being selected among the group consisting of : 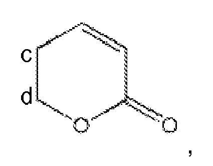 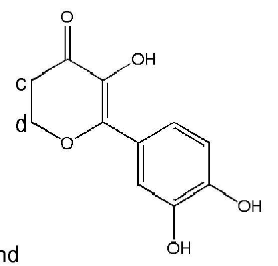 and 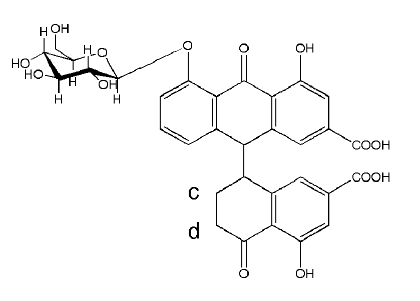 with the provisio that when R1, R2, R4, R5 are H then R3 is not OH,
or any salt thereof, in particular any pharmaceutically acceptable salt thereof.

Preferably, R1, R7, R8, R9 and R10 are H or OH, and R6 is OH or OCH₃. Preferably, when R2 is a moiety of more than 4 atoms, R3 is H, OH, or COCH₃, or when R3 is a moiety of more than 4 atoms, R2 is H, OH, or COCH₃. Preferably, when R2 and R3 together form an heterocycle, then R4 and R5 do not form form an heterocycle; or when R4 and R5 together form an heterocycle, then R2 and R3 do not form form an heterocycle.

In a particular embodiment, R1, R2, R3, R4, R5 can be selected from the above mentioned combinations in Table III.

**Table III : Particular combinations of R1, R2, R3, R4, and R5 of formula (I)**

| R1 | R2 | R3 | R4 | R5 |
|---|---|---|---|---|
| H | 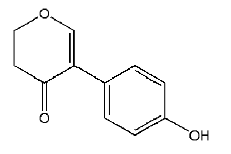 | | H | H |
| H | 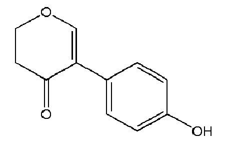 | | OH | H |
| H | 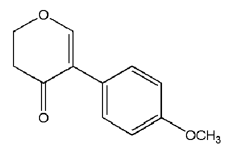 | | H | H |
| H | 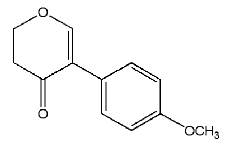 | | OH | H |
| H | 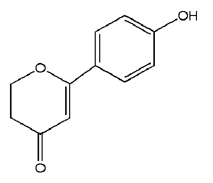 | | OH | H |
| H | 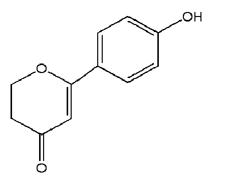 | | H | C-Glucose |
| H | 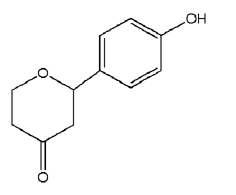 | | OH | H |
| H | 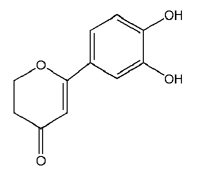 | | OH | H |
| H | 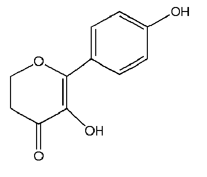 | | OH | H |
| H | 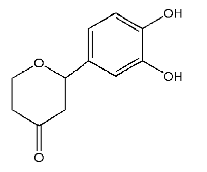 | | OH | H |
| OH | 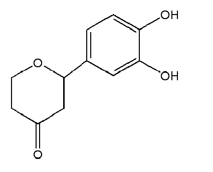 | | H | H |
| H | OH | H | OH | 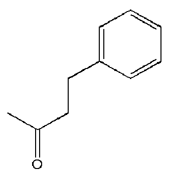 |
| H | OH | H | OH | 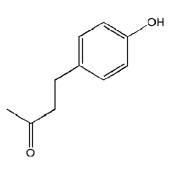 |
| H | H | 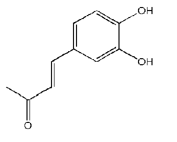 | OH | OH |
| OH | H | OH | 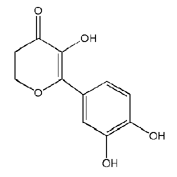 | |
| OH | 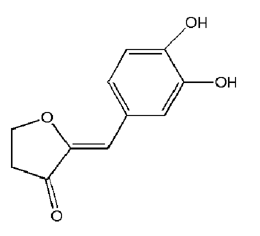 | | H | H |
| H | H | 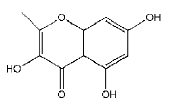 | H | OH |
| H | H | COCH3 | H | H |
| H | H | H | H | COH |
| H | H | H | 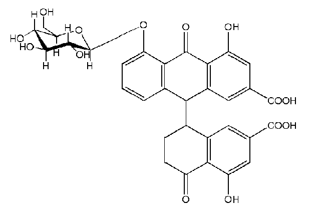 | |
| H | 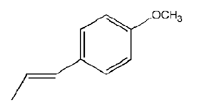 | H | OH | H |
| H | 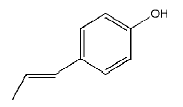 | H | OH | H |
| H | 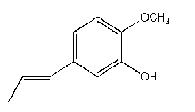 | H | OH | H |
| H | 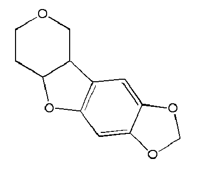 | | H | H |
| H | 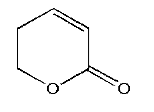 | | H | OH |
| OH | OCH3 | H | 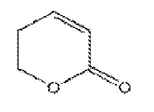 | |

A first preferred [6)-O-α-D-Glcp-(1→]ₙ-6-O-β-D-Glcp-(1→-phenolic derivative of formula (I) with n equal or greater than 1, has R1 and R5 being H; R4 being H or OH; R2 and R3 together forming, when taken together with the atoms to which they are attached, a 6-membered heteroring, substituted by a phenol or a phenoxy, of formula : 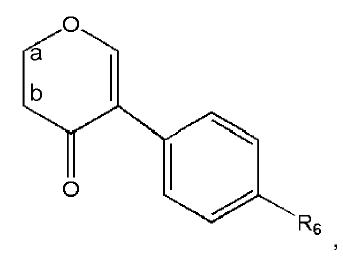 with R6 being OH or O CH₃
and preferably the [6)-O-α-D-Glcp-(1→]ₙ-6-O-β-D-Glcp-(1→-phenolic derivative is selected from the group consisting of [6)-O-α-D-Glcp-(1→]n-6-O⁷-β-D-Glcp-(1→-daidzein (also named [6)-O-α-D-Glcp-(1→]ₙ-daidzin), [6)-O-α-D-Glcp-(1→]n-6-O⁷-β-D-Glcp-(1→-genistein (also named [6)-O-α-D-Glcp-(1→]ₙ-genistin), [6)-O-α-D-Glcp-(1→]ₙ-6-O⁷-β-D-Glcp-(1→-formononetin (also named [6)-α-D-Glcp-(1→]ₙ-ononin), and [6)-O-α-D-Glcp-(1→]n-6-O⁷-β-D-Glcp-(1→-biochanin A (also named [6)-O-α-D-Glcp-(1→]ₙ-sissotrin).

A second preferred [6)-O-α-D-Glcp-(1→]ₙ-6-O-β-D-Glcp-(1→-phenolic derivative of formula (I) with n equal or greater than 1, has R1 being H, R4 being H or OH, R5 being H or 1-C-glucopyranoside, R2 and R3 together forming, when taken with the atoms to which they are attached, a 6-membered heteroring, substituted by a phenol, of formula : 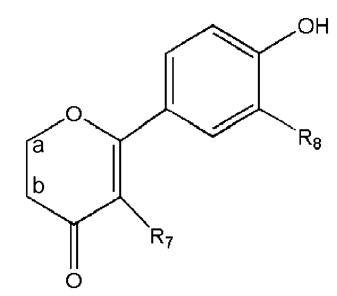 with R7 and R8 being H or OH.

Preferably, R4 is OH and R5 is H, or R4 is H and R5 is 1-C-glucopyranoside. Alternatively, R7 and R8 are both H or one of R7 and R8 is OH and the other is H. More preferably, the [6)-O-α-D-Glcp-(1→]ₙ-6-O-β-D-Glcp-(1→-phenolic derivative is selected from the group consisting of [6)-O-α-D-Glcp-(1→]ₙ-6-O⁷-β-D-Glcp-(1→-apigenin (also named [6)-O-α-D-Glcp-(1→]ₙ-apigetrin), [6)-O-α-D-Glcp-(1→]ₙ-6-O⁷-β-D-Glcp-(1→-isovitexin (also named [6)-O-α-D-Glcp-(1→]ₙ-saponarin), [6)-O-α-D-Glcp-(1→]n-6-O⁷-β-D-Glcp-(1→-luteolin (also named [6)-O-α-D-Glcp-(1→]ₙ-cynaroside), and [6)-O-α-D-Glcp-(1→]n-6-O⁷-β-D-Glcp-(1→-kaempferol (also named [6)-O-α-D-Glcp-(1→]ₙ-populnin).

A third preferred [6)-O-α-D-Glcp-(1→]ₙ-6-O-β-D-Glcp-(1→-phenolic derivative of formula (I) with n equal or greater than 1, has R5 being H, R1 and R4 being H or OH, R2 and R3 together forming, when taken with the atoms to which they are attached, a 6-membered heteroring, substituted by a phenol, of formula : 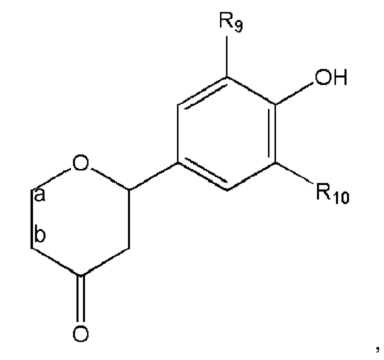 with R9 and R10 being H or OH.

Preferably, R1 and R9 are H, R4 is OH, R10 is H or OH. Alternatively, R4 and R9 are H, R1 is OH, R10 is OH. More preferably, the [6)-O-α-D-Glcp-(1→]ₙ-6-O-β-D-Glcp-(1→-phenolic derivative is selected from the group consisting of [6)-O-α-D-Glcp-(1→]ₙ-6-O⁷-β-D-Glcp-(1→-naringenin (also named [6)-O-α-D-Glcp-(1→]ₙ-prunin), [6)-O-α-D-Glcp-(1→]ₙ-6-O⁷-β-D-Glcp-(1→-eriodictyol (also named [6)-O-α-D-Glcp-(1→]ₙ-pyracanthoside), and [6)-O-α-D-Glcp-(1→]ₙ-6-O⁷-β-D-Glcp-(1→-isookanin (also named [6)-O-α-D-Glcp-(1→]ₙ-flavanomarein).

A fourth preferred [6)-O-α-D-Glcp-(1→]ₙ-6-O-β-D-Glcp-(1→-phenolic derivative of formula (I)with n equal or greater than 1, has R1, R3 and R5 being H, R4 being OH, R2 being 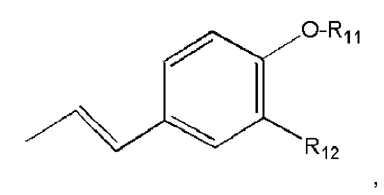 with R11 being H or CH3 and R12 being H or OH.

Preferably, R11 and R12 are H. Alternatively, R11 is CH3 and R12 is H or OH. More preferably the [6)-O-α-D-Glcp-(1→]ₙ-6-O-β-D-Glcp-(1→-phenolic derivative is selected from the group consisting of [6)-O-α-D-Glcp-(1→]ₙ-6-O³-β-D-Glcp-(1→-3,5-dihydroxy-4'-methoxystilbene (also named [6)-O-α-D-Glcp-(1→]ₙ-deoxyrhapontin), [6)-O-α-D-Glcp-(1→]ₙ-6-O³-β-D-Glcp-(1→-resveratrol (also named [6)-O-α-D-Glcp-(1→]ₙ-piceid), and [6)-O-α-D-Glcp-(1→]ₙ-6-O³-β-D-Glcp-(1→-4'-methoxy-3,3',5-stilbenetriol (also named [6)-O-α-D-Glcp-(1→]ₙ-rhapontin).

A fifth preferred [6)-O- α-D-Glcp-(1→]ₙ-6-O-β-D-Glcp-(1→-phenolic derivative of formula (I) with n equal or greater than 1, has R1 and R3 being H, R2 and R4 being OH, and R5 which is 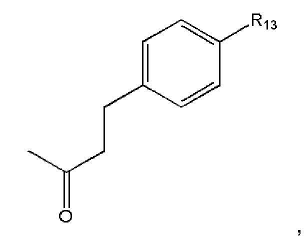 with R13 being H or OH.

Preferably the [6)-O-α-D-Glcp-(1→]ₙ-6-O-β-D-Glcp-(1→-phenolic derivative is selected from the group consisting of [6)-O-α-D-Glcp-(1→]ₙ-6-O^{2'}-β-D-Glcp-(1→-2',4',6'-trihydroxydihydrochalcone (also named [6)-O-α-D-Glcp-(1→]ₙ-2',4',6'-trihydroxydihydrochalcone-2'-O-glucoside or [6)-O-α-D-Glcp-(1→]ₙ-4-deoxyphloridzin), and [6)-O-α-D-Glcp-(1→]ₙ-6-O^{2'}-β-D-Glcp-(1→-phloretin (also named [6)-O-α-D-Glcp-(1→]ₙ-phloridzin).

A sixth preferred [6)-O-α-D-Glcp-(1→]ₙ-6-O-β-D-Glcp-(1→-phenolic derivative of formula (I) with n equal or greater than 1, has R1 and R2 being H, R5 being OH, R4 being H or OH and R3 selected among the group of 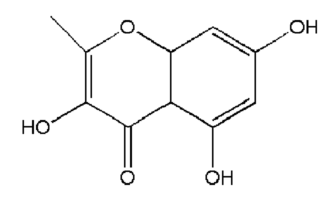 (preferably with R4 being H),
and 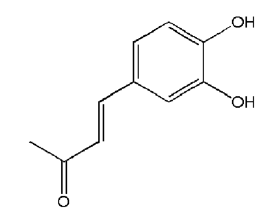 (preferably with R4 being OH).

Preferably, the [6)-O-α-D-Glcp-(1→]ₙ-6-O-β-D-Glcp-(1→-phenolic derivative is selected from the group consisting of [6)-O-α-D-Glcp-(1→]ₙ-6-O^{2'}-β-D-Glcp-(1→-okanin (also named [6)-O-α-D-Glcp-(1→]ₙ-marein), and [6)-O-α-D-Glcp-(1→]ₙ-6-O^{4'}-β-D-Glcp-(1→-quercetin (also named [6)-O-α-D-Glcp-(1→]ₙ-spiraeoside).

A seventh preferred [6)-O-α-D-Glcp-(1→]ₙ-6-O-β-D-Glcp-(1→-phenolic derivative of formula (I) with n equal or greater than 1, has R1, R2 and R4 being H, and R3 and R5 are either respectively COCH₃ and H, or H and COH. Preferably, the [6)-O-α-D-Glcp-(1→]ₙ-6-O-β-D-Glcp-(1→-phenolic derivative is selected from the group consisting of [6)-O-α-D-Glcp-(1→]ₙ-6-O⁴-β-D-Glcp-(1→-4-hydroxyacetophenone (also named [6)-O-α-D-Glcp-(1→]ₙ-picein), and [6)-O-α-D-Glcp-(1→]ₙ-6-O²-β-D-Glcp-(1→-salicylaldehyde (also named [6)-O-α-D-Glcp-(1→]ₙ-helicin).

A eight preferred [6)-O-α-D-Glcp-(1→]ₙ-6-O-β-D-Glcp-(1→-phenolic derivative of formula (I) with n equal or greater than 1, has R4 and R5 being H, R1 is H or OH, R2 and R3 together forming, when taken with the atoms to which they are attached, a heteroring selected from the group consisting of: 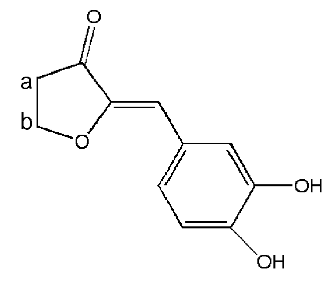 (preferably with R1 being OH),
and 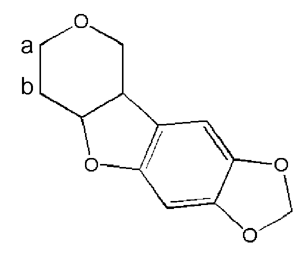 (preferably with R1 being H).

Preferably the [6)-O-α-D-Glcp-(1→]ₙ-6-O-β-D-Glcp-(1→-phenolic derivative is selected from the group consisting of [6)-O-α-D-Glcp-(1→]ₙ-6-O⁶-β-D-Glcp-(1→-maritimetin (also named [6)-O-α-D-Glcp-(1→]ₙ-maritimein), and [6)-O-α-D-Glcp-(1→]ₙ-6-O³-β-D-Glcp-(1→-maackiain (also named [6)-O-α-D-Glcp-(1→]ₙ-trifolirhizin).

A nineth preferred [6)-O-α-D-Glcp-(1→]ₙ-6-O-β-D-Glcp-(1→-phenolic derivative of formula (I) with n equal or greater than 1, has R1 and R4 being H, R5 being OH, and R3 and R4 together forming, when taken with the atoms to which they are attached, a 6-member heteroring of formula :

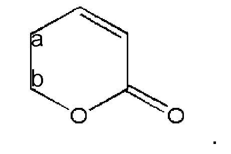

Preferably the [6)-O-α-D-Glcp-(1→]ₙ-6-O-β-D-Glcp-(1→-phenolic derivative is [6)-O-α-D-Glcp-(1→]ₙ-6-O⁶-β-D-Glcp-(1→-esculetin (also named [6)-O-α-D-Glcp-(1→]ₙ-esculin).

A tenth preferred [6)-O-α-D-Glcp-(1→]ₙ-6-O-β-D-Glcp-(1→-phenolic derivative of formula (I) with n equal or greater than 1, has R1 being OH, R2 being OCH3, R3 being H, R4 and R5 together forming, when taken with the atoms to which they are attached, a 6-member heteroring of formula :

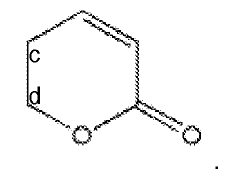

Preferably, the [6)-O-α-D-Glcp-(1→]ₙ-6-O-β-D-Glcp-(1→-phenolic derivative is [6)-O-α-D-Glcp-(1→]ₙ-6-O⁸-β-D-Glcp-(1→-fraxetin (also named [6)-O-α-D-Glcp-(1→]ₙ-fraxin).

A eleventh preferred [6)-O-α-D-Glcp-(1→]ₙ-6-O-β-D-Glcp-(1→-phenolic derivative of formula (I) with n equal or greater than 1, has R1 and R3 being OH, R2 being H, and R4 and R5 together forming, when taken with the atoms to which they are attached, a 6-member heteroring substituted by a phenol of formula :

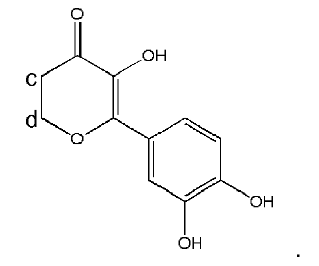

Preferably the [6)-O-α-D-Glcp-(1→]ₙ-6-O-β-D-Glcp-(1→-phenolic derivative is [6)-O-α-D-Glcp-(1→]ₙ-6-O⁸-β-D-Glcp-(1→-gossypetin (also named [6)-O-α-D-Glcp-(1→]ₙ-gossypin).

A twelveth preferred [6)-O-α-D-Glcp-(1→]ₙ-6-O-β-D-Glcp-(1→-phenolic derivative of formula (I) with n equal or greater than 1, has R1 and R2 and R3 being H, R4 and R5 together forming, when taken with the atoms to which they are attached, a cyclohexanone substituted ring of formula :

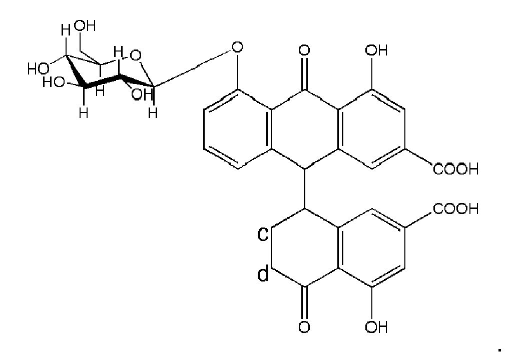

Preferably the [6)-O-α-D-Glcp-(1→]ₙ-6-O-β-D-Glcp-(1→-phenolic derivative is selected from the group consisting of [6)-O-α-D-Glcp-(1→]ₙ-sennoside A and [6)-O-α-D-Glcp-(1→]ₙ-sennoside B.

In a preferred embodiment, the present invention relates to [6)-O-α-D-Glcp-(1→]ₙ-6-O⁷-β-D-Glcp-(1→-daidzein (also named [6)-O-α-D-Glcp-(1→]ₙ-daidzin), [6)-O-α-D-Glcp-(1→]ₙ-6-O⁷-β-D-Glcp-(1→-genistein (also named [6)-O-α-D-Glcp-(1→]ₙ-genistin), [6)-O-α-D-Glcp-(1→]ₙ-6-O⁷-β-D-Glcp-(1→-formononetin (also named [6)-α-D-Glcp-(1→]ₙ-ononin), [6)-O-α-D-Glcp-(1→]ₙ-6-O⁷-β-D-Glcp-(1→-biochanin A (also named [6)-O-α-D-Glcp-(1→]ₙ-sissotrin), [6)-O-α-D-Glcp-(1→]ₙ-6-O⁷-β-O-Glcp-(1→-apigenin (also named [6)-O-α-D-Glcp-(1→]ₙ-apigetrin), [6)-O-α-D-Glcp-(1→]ₙ-6-O⁷-β-D-Glcp-(1→-isovitexin (also named [6)-O-α-D-Glcp-(1→]ₙ-saponarin), [6)-O-α-D-Glcp-(1→]ₙ-6-O⁷-β-D-Glcp-(1→-luteolin (also named [6)-O-α-D-Glcp-(1→]ₙ-cynaroside), [6)-O-α-D-GlCp-(1→]ₙ-6-O⁷-β-D-Glcp-(1→-kaempferol (also named [6)-O-α-D-Glcp-(1→]ₙ-populnin), [6)-O-α-D-Gkcp-(1→]ₙ-6-O⁷-β-D-Glcp-(1→-naringenin (also named [6)-O-α-D-Glcp-(1→]ₙ-prunin), [6)-O-α-D-Glcp-(1→]ₙ-6-O⁷-β-D-Glcp-(1→-eriodictyol (also named [6)-O-α-D-Glcp-(1→]ₙ-pyracanthoside), [6)-O-α-D-Glcp-(1→]ₙ-6-O⁷-β-O-Glcp-(1→-isookanin (also named [6)-O-α-D-Glcp-(1→]ₙ-flavanomarein), [6)-O-α-D-Glcp-(1→]ₙ-6-O³-β-D-Glcp-(1→-3,5-dihydroxy-4'-methoxystilbene (also named [6)-O-α-D-Glcp-(1→]ₙ-deoxyrhapontin), [6)-O-α-D-Glcp-(1→]ₙ-6-O³-β-O-Glcp-(1→-resveratrol (also named [6)-O-α-D-Glcp-(1→]ₙ-piceid), [6)-O-α-D-Glcp-(1→]ₙ-6-O³-β-D-Glcp-(1→-4'-methoxy-3,3',5-stilbenetriol (also named [6)-O-α-D-Glcp-(1→]ₙ-rhapontin), [6)-O-α-D-Glcp-(1→]ₙ-6-O^{2'}-β-D-Glcp-(1→-2',4',6'-trihydroxydihydrochalcone (also named [6)-O-α-D-Glcp-(1→]ₙ-2',4',6'-trihydroxydihydrochalcone-2'-O-glucoside or [6)-O-α-D-Glcp-(1→]ₙ-4-deoxyphloridzin), [6)-O-α-D-Glcp-(1→]ₙ-6-O^{2'}-β-D-Glcp-(1→-phloretin (also named [6)-O-α-D-Glcp-(1→]ₙ-phloridzin), [6)-O-α-D-Glcp-(1→]ₙ-6-O^{2'}-β-D-Glcp-(1→-okanin (also named [6)-O-α-D-Glcp-(1→]ₙ-marein), [6)-O-α-D-Glcp-(1→]ₙ-6-O^{4'}-β-O-Glcp-(1→-quercetin (also named [6)-O-α-D-Glcp-(1→]ₙ**-**spiraeoside), [6)-O-α-D-Glcp-(1→]ₙ-6-O⁴-β-O-Glcp-(1→-4-hydroxyacetophenone (also named [6)-O-α-D-Glcp-(1→]ₙ-picein), [6)-O-α-D-Glcp-(1→]ₙ-6-O²-β-D-Glcp-(1→-salicylaldehyde (also named [6)-O-α-D-Glcp-(1→]ₙ-helicin), [6)-O-α-D-Glcp-(1→]ₙ-6-O⁶-β-O-Glcp-(1→-maritimetin (also named [6)-O-α-D-Glcp-(1→]ₙ-maritimein), [6)-O-α-D-Glcp-(1→]ₙ-6-O³-β-D-Glcp-(1→-maackiain (also named [6)-O-α-D-Glcp-(1→]ₙ-trifolirhizin), [6)-O-α-D-Glcp-(1→]ₙ-6-O⁶-β-O-Glcp-(1→-esculetin (also named [6)-O-α-D-Glcp-(1→]ₙ-esculin), [6)-O-α-D-Glcp-(1→]ₙ-6-O⁸-β-D-Glcp-(1→-fraxetin (also named [6)-O-α-D-Glcp-(1→]ₙ-fraxin), [6)-O-α-D-Glcp-(1→]ₙ-6-O⁸-β-D-Glcp-(1→-gossypetin (also named [6)-O-α-D-Glcp-(1→]ₙ-gossypin), [6)-O-α-D-Glcp-(1→]ₙ-sennoside A and [6)-O-α-D-Glcp-(1→]ₙ-sennoside B, wherein n is egal or greater to 1, more preferably equal to 1.

### Water solubility

The phenolic compounds derivatives of the present invention have an improved solubility in water of at least 50% to 5000% as compared to the corresponding O-β-D-Glcp-(1→)-phenolic compound in the same physiological conditions, more preferably at least 50%, 100%, 200%, 400%, 800%, 1000%, 2000%, 3000%, 4000% or 5000%. Solubility of a phenolic compound derivative can be estimated by preparing a saturated water solution of said phenolic compound derivative. Excess solid form of phenolic compound derivative is eliminated by high speed centrifugation and concentration of compound A in the supernatant is estimated by HPLC using appropriate references (see above § 'Prepation of derivative' for HPLC conditions example). Solubility difference is calculated with formula :
%solubility difference = (A-B)/A x 100
wherein
A is water solubility of [6)-O-α-D-Glcp-(1→]ₙ-6-O-β-D-Glcp-(1→-phenolic derivative, and
B is water solubility of O-β-D-Glcp-(1→)-phenolic compound

In a preferred embodiment, phenolic compounds derivatives of the present invention have an improved solubility in water of at least 50% as compared to the corresponding O-β-D-Glcp-(1→)-phenolic derivative in the same physiological conditions.

The present invention contemplates the salts of the [6)-O-α-D-Glcp-(1→]ₙ-6-O-β-D-Glcp-(1→-phenolic derivatives in particular the pharmaceutically acceptable salts thereof.

Such salts include pharmaceutically acceptable acid addition salts, pharmaceutically acceptable base addition salts, pharmaceutically acceptable metal salts, ammonium and alkylated ammonium salts. Acid addition salts include salts of inorganic acids as well as organic acids. Representative examples of suitable inorganic acids include hydrochloric, hydrobromic, hydroiodic, phosphoric, sulfuric, perchloric, and the like. Representative examples of suitable organic acids include formic, acetic, trichloroacetic, trifluoroacetic, propionic, benzoic, cinnamic, citric, fumaric, and the like. Further examples of pharmaceutically acceptable inorganic or organic acid addition salts include the pharmaceutically acceptable salts listed in J. Pharm. Sci. 1977, 66, 2, and in Handbook of Pharmaceutical Salts: Properties, Selection, and Use edited by P. Heinrich Stahl and Camille G. Wermuth 2002. Examples of metal salts include lithium, sodium, potassium, magnesium salts and the like. Examples of ammonium and alkylated ammonium salts include ammonium, methylammonium, dimethylammonium, trimethylammonium, ethylammonium, hydroxyethylammonium, diethylammonium, butylammonium, tetramethylammonium salts and the like. Examples of organic bases include lysine, arginine, guanidine, diethanolamineoline and the like.

### in situ release properties

Surprisingly, the inventors found that the [6)-O-α-D-Glcp-(1→]ₙ-6-O-β-D-Glcp-(1→-phenolic derivatives of the present invention can be cleaved by enzymes (glycosidases such as an amylase and/or a pullulanase and/or a α-glucosidase and/or β-glucosidase) leading to *in situ* release of the corresponding aglycone or [6)-O-α-D-Gicp-(1→]ₙ₋ₓ-6-O-β-D-Glcp-(1→-phenolic derivative (wherein x=1 to n).

All the [6)-O-α-D-Glcp-(1→]ₙ-6-O-β-D-Glcp-(1→-phenolic derivatives of the present invention have at least one O-α-glucoside bond. This bond can be specifically hydrolyzed by enzymes, such α-glucosidases (EC 3.2.1.20), to release [6)-O-α-D-Glcp-(1→]ₙ₋ₓ-6-O-β-D-Glcp-(1→-phenolic derivative (wherein x=1 to n). For x=n, the beta-glucoside can be further cleaved by β-glucosidases to release the aglycone part from glucose.

When achieved *in situ*, this liberation has several advantages:
- it enables to release the poorly soluble aglycone (which may be more active than the glycoside derivative) after their administration/injection/application under a soluble glycoside form, and/or
- the in situ release can be time dependant (if achieved by enzymes expressed by micro-organims, the amount of releasing enzyme will be correlated to the number of micro-organisms : the more dense the bacterial population will be, the more aglycones release will occur), and/or
- the *in situ* release can be controlled by an *in situ* administration/injection/application of appropriate mix of glycosidases or of a micro-organism expressing such enzymatic activity, and/or
- the *in situ* release is time controlled and can depends on the value of n : the higher is n, the slowest the release is by exo-glycosidases.

These advantages are important in the formulation of phenolics in cosmetics, dermocosmetics, nutritional or healthcare preparations.

It has been also shown that skin microflora has a natural alpha-glucosidase activity (see PCT/EP07/055815) and that there are enzymes which enable the deglycosylation of β-glucoside-phenolic compounds in human gut (see the example of Genistein-7-*O-*glucoside and Daidzein-7-*O*-glucoside in Scalbert et al., 2000, Journal of Nutrition, Dietary Intake and Bioavailability of Polyphenols).

In a preferred embodiment of the present invention, the [6)-O-α-D-Glcp-(1→]ₙ-6-O-β-D-Glcp-(1→-phenolic derivatives can be *in situ* hydrolyzed by enzyme(s) expressed by human cells, and/or human associated micro-organisms, and more preferentially by human skin associated micro-organisms. Known and non exhaustive examples of such human commensal or non commensal micro-organisms include *Streptococcus* species, *Staphylococcus* species, *Enterococcus* species, *Escherichia coli, Bacilli, Corynebacterium* species, *Propionibacterium* species.

When applied on skin, [6)-O-α-D-Glcp-(1→]ₙ-6-O-β-D-Glcp-(1→-phenolic derivatives of the present invention can therefore be converted by skin associated micro-organisms into the aglycones part and the glucosyl and/or isomaltosyl residue. Such bacteria can be found in human beings in mouth, intestinal tract, genital tract and upper respiratory system.

Therefore the present invention also relates to [6)-O-α-D-Glcp-(1→]ₙ-6-O-β-D-Glcp-(1→-phenolic derivative which enable the *in situ* slow release of the corresponding aglycone or [6)-O-α-D-Glcp-(1→]ₙ₋ₓ-6-O-β-D-Glcp-(1→-phenolic derivative (wherein x=1 to n) through their hydrolysis by a glycosidase of human cells, or of natural microbiotes, and more specifically of human skin micro-organisms. Said slow release of the aglycone or of [6)-O-α-D-Glcp-(1→]ₙ₋ₓ-6-O-β-D-Glcp-(1→-phenolic derivative (wherein x=1 to n) forms are of great interest for dermatology, cosmetics, healthcare and nutrition.

In a much preffered embodiment of the present invention, the [6)-O-α-D-Glcp-(1→]ₙ-6-O-β-D-Glcp-(1→-phenolic derivative, wherein n=1, enable the *in situ* slow release of the corresponding aglycone or O-β-D-Glcp-(1→-phenolic derivative through their hydrolysis by a glycosidase of human cells, or of natural microbiotes, and more specifically of human skin micro-organisms.

In a preferred embodinment of the invention, the *in situ* release of the [6)-O-α-D-Glcp-(1→]ₙ-6-O-β-D-Glcp-(1→-phenolic derivative generates an isomaltoside which has been shown to have interesting biological properties (Patent JP8214895 : Caries suppressing agent, its production and use thereof).

Therefore the present invention also relates to [6)-O-α-D-Glcp-(1→]ₙ-6-O-β-Glcp-(1→-phenolic derivative which enable the *in situ* slow release of isomaltoside through their hydrolysis by a glycosidase of human cells, or of natural microbiotes, and more specifically of human skin micro-organisms.

In a much preferred embodiment of the invention, the [6)-O-α-D-Glcp-(1→]ₙ-6-O-β-D-Glcp-(1→-phenolic derivative, wherein n=1, enable the *in situ* slow release of isomaltose through their hydrolysis by a glycosidase of human cells, or of natural microbiotes, and more specifically of human skin micro-organisms.

In a futher preferred embodiment, the present invention relates to [6)-O-α-D-Glcp-(1→]ₙ-6-O-β-D-Glcp-(1→]ₙ-phenolic derivative, wherein n=1, selected among the column A of table below, which enable the in situ slow release of compounds listed on the same line in column B and/or C of Table IV below :

**TABLE IV : Hydrolysis products of some [6)-O-α-D-Glcp-(1→]ₙ-6-O-β-D-Glcp-(1→-phenolic derivatives**

| A (diglucosylated form) | B (beta-glucoside form) | C (aglycone form) |
|---|---|---|
| O-α-D-Glcp-(→⁶)-O⁷-β-D-Glcp-(1→-daidzein also named O-α-D-Glcp-(1→6)-daidzin | O⁷-β-D-Glcp-(1→-daidzein also named daidzein-7-O-β-D-glucopyranoside or daidzin | daidzein |
| O-α-D-Glcp-(1→6)-O⁷-β-D-Glcp-(1→-genistein also named O-α-D-Glcp-(1→6)-genistin | O⁷-β-D-Glcp-(1→-genistein also named genistein-7-O-β-D-glucopyranoside or genistin | genistein |
| O-α-D-Glcp-(1→6)-O⁷-D-Glcp-(1→- formononetin also named O-α-D-Glcp-(1→6)-ononin | O⁷-β-D-Glcp-(1→-formononetin also named formononetin-7-O-β-D-glucopyranoside or ononin | formononetin |
| O-α-D-Glcp-(1→6)-O⁷-β-D-Glcp-(1→-biochanin A also named O-α-D-Glcp-(1→6)-sissotrin | O⁷-β-D-Glcp-(1→-biochanin A also named biochanin A-7-O-β-D-glucopyranoside or sissotrin | sissotrin |
| O-α-D-Glcp-(1→6)-O⁷-β-D-Glcp-(1→-apigenin also named O-α-D-Glcp-(1→6)-apigetrin | O⁷-β-D-Glcp-(1→-apigenin also named apigenin-7-O-β-D-glucopyranoside or apigetrin | apigenin |
| O-α-D-Glcp-(1→6)-O⁷-β-O-Glcp-(1→-isovitexin also named O-α-D-Glcp-(1→6)-saponarin | O⁷-β-D-Glcp-(1→-isovitexin also named isovitexin-7-O-β-D-glucopyranoside or saponarin | isovitexin |
| O-α-D-Glcp-(1→6)-O⁷-β-O-Glcp-(1→-naringenin also named O-α-D-Glcp-(1→6)-prunin | O⁷-β-D-Glcp-(1→-naringenin also named naringenin-7-β-D-glucopyranoside or prunin | naringenin |
| O-α-D-Glcp-(1→6)-O⁷-β-D-Glcp-(1→-luteolin also named O-α-D-Glcp-(1→6)-cynaroside | O⁷-β-O-Glcp-(1→-luteolin also named luteolin-7-O-β-D-glucopyranoside or cynaroside | luteolin |
| O-α-D-Glcp-(1→6)-O⁷-β-D-Glcp-(1→-kaempferol also named O-α-D-Glcp-(1→6)-populnin | O⁷-β-D-Glcp-(1 →-kaempferol also named kaempferol-7-O-β-D-glucopyranoside or populnin | kaempferol |
| O-α-D-Glcp-(1→6)-O⁷-β-O-Glcp-(1→-7-eriodictyol also named O-α-D-Glcp-(1→6)-pyracanthoside | O⁷-β-D-Glcp-(1→-eriodictyol also named eriodictyol-7-O-β-D-glucopyranoside or pyracanthoside | eriodictyol |
| O-α-D-Glcp-(1→6)-O⁷-β-O-Glcp-(1→-isookanin also named O-α-D-Glcp-(1→6)-flavanomarein | O⁷-β-D-Glcp-(1→-isookanin also named isookanin-7-O-β-D-glucopyranoside or flavanomarein | isookanin |
| O-α-D-Glcp-(1→6)-O²-β-D-Glcp-(1→-2',4',6'-trihydroxydihydrochalcone | O²'-β-D-Glcp-(1→2',4',6'-trihydroxydihydrochalcone also named 2',4',6'- | 2',4',6'-trihydroxydihydroch alcone |
| also named O-α-D-Glcp-(1→6)-2',4',6'-trihydroxydihydrochalcone-2'-O-glucoside or α-D-Glcp-(1→6)-4-deoxyphloridzin | trihydroxydihydrochalcone-2'-O-β-D-glucopyranoside or 4-deoxyphloridzin | |
| O-α-D-Glcp-(1→6)-O^{2'}-β-D-Glcp-(1→-phloretin also named O-α-D-Glcp-(1→6)- phloridzin | O^{2'}-β-D-Glcp-(1→-phloretin also named phloretin-2'-O-β-D-glucopyranoside or phloridzin | phloretin |
| O-α-D-Glcp-(1→6)-O^{4'}-δ-D-Glcp-(1→-okanin also named O-α-D-Glcp-(1→6)-marein | O^{4'}-β-D-Glcp-(1→-okanin also named okanin-4'-O-β-D-glucopyranoside or marein | okanin |
| O-α-D-Glcp-(1→6)-O⁸-β-D-Glcp-(1→-gossypetin also named O-α-D-Glcp-(1→6)-gossypin | O⁸-β-O-Glcp-(1→-gossypetin also named gossypetin-8-O-β-D-glucopyranoside or gossypin | gossypin |
| O-α-D-Glcp-(1→6)-O⁶-β-D-Glcp-(1→-maritimetin also named O-α-D-Glcp-(1→6)-maritimein | O⁸-β-D-Glcp-(1→-maritimein also named maritimetin-6-O-β-D-glucopyranoside or maritimein | maritimetin |
| O-α-D-Glcp-(1→6)-O^{4'}-β-D-Glcp-(1→-quercetin also named O-α-D-Glcp-(1→6)-spiraeoside | O^{4'}-β-D-Glcp-(1→-quercetin also named quercetin-4'-O-β-D-glucopyranoside or spiraeoside | quercetin |
| O-α-D-Glcp-(1→6)-O²-β-D-Glcp-(1→-salicylaldehyde also named O-α-D-Glcp-(1→6)-helicin | O²-β-D-Glcp-(1→-salicylaldehyde also named salicylaldehyde-O-β-D-glucopyranoside or helicin | salicylaldehyde |
| O-α-D-Glcp-(1→6)-sennoside A or B | sennoside A or B | sennidin A or B |
| O-α-D-Glcp-(1→6)-O³-β-D-Glcp-(1→-3,5-dihydroxy-4'-methoxystilbene also named O-α-D-Glcp-(1→6)-deoxyrhapontin | O'-β-D-Glcp-(1-3,5-dihydroxy-4'-methoxystilbene also named 3,5-dihydroxy-4'-methoxystilbene-3-O-β-D-glucopyranoside or deoxyrhapontin | 3,5-dihydroxy-4'-methoxystilbene |
| O-α-D-Glcp-(1→6)-O³-β-D-Glcp-(1→-resveratrol also named O-α-D-Glcp-(1→6)-piceid | O³-β-D-Glcp-(1→-resveratrol also named resveratrol-3-O-β-D-glucopyranoside or piceid | resveratrol |
| O-α-D-Glcp-(1→6)-O³-β-D-Glcp-(1→-4'-methoxy-3,3',5-stilbenetriol also named O-α-D-Glcp-(1→6)-rhapontin | O³-β-D-Glcp-(1→-4'-methoxy-3,3',5-stilbenetriol also named 4'-methoxy-3,3',5-stilbenetriol-3-O-β-D-glucopyranoside or rhapontin | 4'-methoxy-3,3',5-stilbenetriol |
| O-α-D-Glcp-(1→6)-O³-β-D-Glcp-(1→-maackiain also named O-α-D-Glcp-(1→6)-trifolirhizin | O³-β-D-Glcp-(1→-maackiain also named maackiain-3-O-β-D-glucopyranoside or trifolirhizin | maackiain |
| O-α-D-Glcp-(1→6)-O⁶-β-D-Glcp-(1→-esculetin also named O-α-D-Glcp-(1→6)-esculin | O⁶-β-D-Glcp-(1→-esculetin also named esculetin-6-O-β-D-glucopyranoside or esculin | esculetin |
| O-α-D-Glcp-(1→6)-O⁴-β-D-Glcp-(1→-4-hydroxyacetophenone also named O-α-D-Glcp-(1→6)-picein | O⁴-β-D-Glcp-(1→-4-hydroxyacetophenone also named 4-hydroxyacetophenone-4-O-β-D-glucopyranoside or picein | 4-hydroxyacetopheno ne |
| O-α-D-Glcp-(1→6)-O⁸-β-O-Glcp-(1→-fraxetin also named O-α-D-Glcp-(1→6)-fraxin | O⁸-β-O-Glcp-(1→-fraxetin also named fraxetin-8-O-β-D-glucopyranoside or fraxin | fraxetin |

Therefore, the present invention concerns a pharmaceutical, nutritional, dermatological or cosmetic composition comprising a [6)-O-α-D-Glcp-(1→]ₙ-6-O-β-D-Glcp-(1→-phenolic derivative of the present invention or a salt thereof, in particular a pharmaceutically, nutritionally or cosmetically acceptable sat thereof. The present invention also concerns a [6)-O-α-D-Glcp-(1→]ₙ-6-O-β-D-Glcp-(1→-phenolic derivative of the present invention or a pharmaceutically acceptable salt thereof as a medicament. The medicament can be therapeutic or prophylactic. [6)-O-α-D-Glcp-(1→]n-6-O-β-D-Glcp-(1→-phenolic derivative of the present invention have several activity among which antiviral, antibacterial, immune-stimulating, antiallergic, antihypertensive, antiischemic, antiarrhytmic, antithrombotic, hypocholesterolemic, antidiabete, antilipoperoxidant, hepatoprotective, anti-inflammatory, anticarcinogenic antimutagenic, antineoplastic, anti-thrombotic, and vasodilatory actions. Accordingly, the present invention relates to a [6)-O-α-D-Glcp-(1→]ₙ-6-O-β-D-Glcp-(1→-phenolic derivative of the present invention or a pharmaceutically acceptable salt thereof as antiviral, antibacterial, immune-stimulating, antiallergic, antihypertensive, antiischemic, antiarrhytmic, antithrombotic, hypocholesterolemic, antidiabete, antilipoperoxidant, hepatoprotective, anti-inflammatory, anticarcinogenic antimutagenic, antineoplastic, anti-thrombotic, and vasodilatory agent. It also relates to the use of a [6)-O-α-D-Glcp-(1→]ₙ-6-O-β-D-Glcp-(1→-phenolic derivative of the present invention or a pharmaceutically acceptable salt thereof for the preparation of antiviral, antibacterial, immune-stimulating, antiallergic, antihypertensive, antiischemic, antiarrhytmic, antithrombotic, hypocholesterolemic, antidiabete, antilipoperoxidant, hepatoprotective, anti-inflammatory, anticarcinogenic antimutagenic, antineoplastic, anti-thrombotic, and vasodilatory agent. The [6)-O-α-D-Glcp-(1→]ₙ-6-O-β-D-Glcp-(1→-phenolic derivative of the present invention can be considered as a pro-drug allowing the in situ release of the drug.

In a particular embodiment, the composition can further comprise a glycosidase or a micro-organism expressing said glycosidase activity. Preferably, the glycosidase or a micro-organism expressing said glycosidase activity is present in the composition in an inactivated form and the glycosidase is activated just at the moment of administration.

For instance, the composition can be formulated in dried form, the absence of water leading to the inactivation of glycosidase; after water addition, the enzyme will become active and will be then able to hydrolyze the glucosidic bond. The enzyme and the [6)-O-α-D-Glcp-(1→]ₙ-6-O-β-D-Glcp-(1→-phenolic derivatives can be put in two different liquid preparations that will be mixed just at the moment of administration. If the enzyme and the [6)-O-α-D-Gicp-(1→]ₙ-6-O-β-D-Glcp-(1→-phenolic derivatives are put into the same solution, it is possible to use an enzyme reversible inhibitor that will be diluted after administration, thus allowing the enzyme to recover its ability to hydrolyze the [6)-O-α-D-Glcp-(1→]ₙ-6-O-β-D-Glcp-(1→-phenolic derivatives. [6)-O-α-D-Glcp-(1→]ₙ-6-O-β-D-Glcp-(1→-phenolic derivatives of the present invention and the glycosidase or a micro-organism expressing said glycosidase activity can also be physically separated (e.g., microcapsule). The present invention relates to a product containing a [6)-O-α-D-Glcp-(1→]ₙ-6-O-β-D-Glcp-(1→-phenolic derivative of the present invention or a pharmaceutically acceptable salt thereof and a glycosidase or a micro-organism expressing said glycosidase activity as a combined preparation for simultaneous, separate or sequential use.

The present invention concerns the use of a [6)-O-α-D-Glcp-(1→]ₙ-6-O-β-D-Glcp-(1→-phenolic derivative of the present invention or a pharmaceutically acceptable salt thereof for preparing a pharmaceutical composition for treating or preventing a cancer, a cardiovascular disease, a bacterial infection, a viral infection, a fungal infection, a UV-induced erythema, an allergy, a metabolism disorder, diabetes, an obesity, an hormonal disorder, a bone disease, a pain, a brain disease, a mouth or teeth disease, an inflammatory or immune disorder.

In particular, the cancer is a solid tumor, for example a breast or colon cancer. In particular, the allergy can be allergic rhinoconjunctivitis. Therefore, the present invention also concerns a method for treating or preventing a cancer, a cardiovascular disease, a bacterial infection, a viral infection, a fungal infection, a UV-induced erythema, an allergy, a metabolism disorder, diabetes, an obesity, an hormonal disorder, a bone disease, a pain, a brain disease, a mouth or teeth disease, an inflammatory or immune disorder comprising administering a [6)-O-α-D-Glcp-(1→]ₙ-6-O-β-D-Glcp-(1→-phenolic derivative of the present invention or a pharmaceutically acceptable salt thereof. In addition, the method can further comprise the step of administering sequentially or simultaneously a glycosidase or a micro-organism expressing said glycosidase activity. Preferably, the glycosidase or a micro-organism expressing said glycosidase activity is administered by the same route.

In a particular embodiment, the present invention concerns the use of a [6)-O-α-D-Glcp-(1→]ₙ-6-O-β-D-Glcp-(1→-phenolic derivative of the present invention or a pharmaceutically or cosmetically acceptable salt thereof for preparing a pharmaceutical, dermatological or cosmetic composition to be administered topically (i.e., on skin), wherein enzymes issued from skin associated micro-organisms release the corresponding aglycone of the [6)-O-α-D-Glcp-(1→]ₙ-6-O-β-D-Glcp-(1→-phenolic derivative. In addition, the present invention concerns the use of a [6)-O-α-D-Glcp-(1→4]ₙ-6-O-β-D-Glcp-(1→-phenolic derivative of the present invention for preparing a pharmaceutical or cosmetic composition to be administered orally, wherein enzymes issued from mouth and intestinal tract associated micro-organisms release the corresponding aglycone of the [6)-O-α-D-Glcp-(1→]ₙ-6-O-β-D-Glcp-(1→-phenolic derivative. The present invention also concerns the use of a [6)-O-α-D-Glcp-(1→]ₙ-6-O-β-D-Glcp-(1→-phenolic derivative of the present invention for preparing a pharmaceutical or cosmetic composition to be administered rectally, wherein enzymes issued from intestinal tract associated micro-organisms release the corresponding aglycone of the [6)-O-α-D-Glcp-(1→]ₙ-6-O-β-D-Glcp-(1→-phenolic derivative. The present invention further concerns the use of a [6)-O-α-D-Glcp-(1→]ₙ-6-O-β-D-Glcp-(1→-phenolic derivative of the present invention for preparing a pharmaceutical or cosmetic composition to be administered nasally, wherein enzymes issued from upper respiratory system associated micro-organisms release the corresponding aglycone of the [6)-O-α-D-Glcp-(1→]ₙ-6-O-β-D-Glcp-(1→-phenolic derivative. The present invention further concerns the use of a [6)-O-α-D-Glcp-(1→]ₙ-6-O-β-D-Glcp-(1→-phenolic derivative of present invention for preparing a pharmaceutical or cosmetic composition to be administered vaginally, wherein enzymes issued from female genital tract associated micro-organisms release the corresponding aglycone of the [6)-O-α-D-Glcp-(1→]ₙ-6-O-β-D-Glcp-(1→-phenolic derivative.

The present invention also concerns a combination of a [6)-O-α-D-Glcp-(1→]ₙ-6-O-β-Glcp-(1→-phenolic derivatives of the present invention or a pharmaceutically acceptable salt thereof with a glycosidase or a micro-organism expressing said glycosidase activity for a simultaneous or sequential administration. When simultaneously administration is performed, the [6)-O-α-D-Glcp-(1→]ₙ-6-O-β-D-Clcp-(1→-phenolic derivatives of the present invention or a pharmaceutically acceptable salt thereof and the glycosidase or a micro-organism expressing said glycosidase activity can be administereted in the same or different compositions.

In particular, the [6)-O-α-D-Glcp-(1→]ₙ-6-O-β-D-Glcp-(1→-phenolic derivatives of interest in cosmetic field are those having softening properties, anti- inflammatory properties, antipruritic properties, antiseptic properties, antiperspirant properties, soothing properties, healing properties, stimulating properties, properties promoting the support of the blood and lymphatic microcirculation, texturing properties, antioxidant properties, foaming or emulsifying properties, photoprotective properties, thickening or absorbent properties, and/or odorous properties. For instance, the following [6)-O-α-D-Glcp-(1→]ₙ-6-O-β-D-Glcp-(1→-phenolic derivatives of the present invention have an interest in cosmetic field :
- [6)-O-α-D-Glcp-(1→]ₙ-6-O⁷-β-D-Glcp-(1→-genistein (also named [6)-O-α-D-Glcp-(1→]ₙ-genistin) as UV-protective agent;
- [6)-O-α-D-Glcp-(1→]ₙ-6-O⁷-β-D-Glcp-(1→-apigenin (also named [6)-O-α-D-Glcp-(1→]ₙ-apigetrin) as antioxidant agent;
- [6)-O-α-D-Glcp-(1→]ₙ-6-O⁷-β-D-Glcp-(1→-isovitexin (also named [6)-O-α-D-Glcp-(1→]ₙ-saponarin) as antioxidant or antibacterial agent;
- [6)-O-α-D-Glcp-(1→]ₙ-6-O⁷-β-D-Glcp-(1→naringenin (also named [6)-O-α-D-Glcp-(1→]ₙprunin), [6)-O-α-D-Glcp-(1→]ₙ-6-O⁷-β-D-Glcp-(1→-kaempferol (also named [6)-O-α-D-Glcp-(1→]ₙ-populnin), [6)-O-α-D-Glcp-(1→]ₙ-6-O⁷-β-D-Glcp-(1→-eriodictyol (also named [6)-O-α-D-Glcp-(1→]ₙ-pyracanthoside), [6)-O-α-D-Glcp-(1→]ₙ-6-O⁷-β-D-Glcp-(1→-isookanin (also named [6)-O-α-D-Glcp-(1→]ₙ-flavanomarein), [6)-O-α-D-Glcp-(1→]ₙ-6-O^{2'}-β-D-Glcp-(1→-2',4',6'-trihydroxydihydrochalcone (also named [6)-O-α-D-Glcp-(1→]ₙ-2',4',6'-trihydroxydihydrochalcone-2'-O-glucoside or [6)-O-α-D-Glcp-(1→]ₙ-4-deoxyphloridzin), [6)-O-α-D-Glcp-(1→]ₙ-6-O^{2'}-β-D-Glcp-(1→-okanin (also named [6)-O-α-D-Glcp-(1→]ₙ-marein), [6)-O-α-D-Glcp-(1→]ₙ-6-O⁶-β-D-Glcp-(1→-maritimetin (also named [6)-O-α-D-Glcp-(1→]ₙ-maritimein), [6)-O-α-D-Glcp-(1→]ₙ-6-O^{4'}-β-D-Glcp-(1→-quercetin (also named [6)-O-α-D-Glcp-(1→]ₙ-spiraeoside), [6)-O-α-D-Glcp-(1→]ₙ-6-O³-β-D-Glcp-(1→-resveratrol (also named [6)-O-α-D-Glcp-(1→]ₙ-piceid), and [6)-O-α-D-Glcp-(1→]ₙ-6-O⁸-β-D-Glcp-(1→-fraxetin (also named [6)-O-α-D-Glcp-(1→]ₙ-fraxin) as antioxidant agent;
- [6)-O-α-D-Glcp-(1→]ₙ-6-O⁷-β-D-Glcp-(1→-luteolin (also named [6)-O-α-D-Glcp-(1→]ₙ-cynaroside) as antibacterial agent;
- [6)-O-α-D-Glcp-(1→]ₙ-6-O³-β-D-Glcp-(1→-maackiain (also named [6)-O-α-D-Glcp-(1→]ₙ-trifolirhizin) as a skin whitening agent;
- [6)-O-α-D-Glcp-(1→]ₙ-6-O^{2'}-β-D-Glcp-(1→-phloretin (also named [6)-O-α-D-Glcp-(1→]ₙ-phloridzin) as antioxidant, anti-inflammatory, and/or promenalogenic agent;
- [6)-O-α-D-Glcp-(1→]ₙ-6-O⁷-β-D-Glcp-(1→-daidzein (also named [6)-O-α-D-Glcp-(1→]ₙ-daidzin) as antiallergic and/or anti-inflammatory agent;
- [6)-O-α-D-Glcp-(1→4]ₙ-6-O³-β-D-Glcp-(1→-3,5-dihydroxy-4'-methoxystilbene (also named [6)-O-α-D-Glcp-(1→]ₙ-deoxyrhapontin) as antioxidant and/or anti-inflammatory agent; and,
- [6)-O-α-D-Glcp-(1→]ₙ-6-O³-β-D-Glcp-(1→-resveratrol (also named [6)-O-α-D-Glcp-(1→]ₙ-piceid) as antioxidant, anti-aging and/or anti-inflammatory agent.

For instance, the following [6)-α-D-Glcp-(1→]ₙ-6-O-β-D-Glcp-(1→-phenolic derivatives of the present invention have an interest in therapeutic field :
- [6)-O-α-D-Glcp-(1→]ₙ-6-O⁷-β-β-Glcp-(1→-daidzein (also named [6)-O-α-D-Glcp-(1→]ₙ-daidzin) as antiallergic and antithrombotic agent or for preventing osteonecrosis, therefore useful for treating or preventing cardiovascular disease, an allergy, an inflammatory disease or a bone disease;
- [6)-O-α-D-Glcp-(1→]ₙ-6-O⁷-β-D-Glcp-(1→-genistein (also named [6)-O-α-D-Glcp-(1→]ₙ-genistin) as UV-protective agent and for reducing bone loss; therefore useful for treating or preventing a UV-induced erythema, or a bone disease:

- [6)-O-α-D-Glcp-(1→]ₙ-6-O⁷-β-D-Glcp-(1→-formononetin (also named [6)-α-D-Glcp-(1→]ₙ-ononin) as cell protective agent; therefore useful for treating or preventing a brain disease such as a neurodegenerative disease;
- [6)-O-α-D-Glcp-(1→]ₙ-6-O⁷-β-D-Glcp-(1→-biochanin A (also named [6)-O-α-D-Glcp-(1→]ₙ-sissotrin) as glucose regulation agent; therefore useful for treating or preventing a metabolism disorder, diabetes or the obesity;
- [6)-O-α-D-Glcp-(1→]ₙ-6-O⁷-β-D-Glcp-(1→-apigenin (also named [6)-O-α-D-Glcp-(1→]ₙ-apigetrin) as antioxidant and antimicrobial agent; therefore useful for treating or preventing a bacterial, viral or fungal infection, a cancer, or a brain disease;
- [6)-O-α-D-Glcp-(1→]ₙ-6-O⁷-β-D-Glcp-(1-isovitexin (also named [6)-O-α-D-Glcp-(1→]ₙ-saponarin) as antioxidant or antibacterial agent; therefore useful for treating or preventing a bacterial infection, a cancer, or a brain disease;
- [6)-O-α-D-Glcp-(1→]ₙ-6-O⁷-β-D-Glcp-(1→-naringenin (also named [6)-O-α-D-Glcp-(1→]ₙ-prunin), [6)-O-α-D-Glcp-(1→]ₙ-6-O⁷-β-D-Glcp-(1→-kaempferol (also named [6)-O-α-D-Glcp-(1→]ₙ-populnin), [6)-O-α-D-Glcp-(1→]ₙ-6-O⁷-β-D-Glcp-(1→-eriodictyol (also named [6)-O-α-D-Glcp-(1→]ₙ-pyracanthoside), [6)-O-α-D-Glcp-(1→]n-6-O⁷-D-Glcp-(1→-isookanin (also named [6)-O-α-D-Glcp-(1→]ₙ-flavanomarein), [6)-O-α-D-Glcp-(1→]ₙ-6-O^{2'}-β-D-Glcp-(1→-2',4',6'-trihydroxydihydrochalcone (also named [6)-O-α-D-Glcp-(1→]ₙ-2',4',6'-trihydroxydihydrochalcone-2'-O-glucoside or [6)-O-α-D-Glcp-(1→]ₙ-4-deoxyphloridzin), [6)-O-α-D-Glcp-(1→]ₙ-6-O^{2'}-β-D-Glcp-(1→-okanin (also named [6)-O-α-D-Glcp-(1→]ₙ-marein), [6)-O-α-D-Glcp-(1→]ₙ-6-O⁶-β-D-Glcp-(1→-maritimetin (also named [6)-O-α-D-Glcp-(1→]ₙ-maritimein), and [6)-O-α-D-Glcp-(1→]ₙ-6-O⁸-β-D-Glcp-(1→-fraxetin (also named [6)-O-α-D-Glp-(1→]ₙ-fraxin) as antioxidant agent; therefore useful for treating or preventing a cancer, or a brain disease;
- [6)-O-α-D-Glcp-(1→]ₙ-6-O⁷-β-D-Glcp-(1→-luteolin (also named [6)-O-α-D-Glcp-(1→]ₙ-cynaroside) as antibacterial or antiproliferative agent; therefore useful for treating or preventing a bacterial infection, or a cancer;
- [6)-O-α-D-Glcp-(1→]ₙ-6-O^{2'}-β-D-Glcp-(1→-phloretin (also named [6)-O-α-D-Glcp-(1→]ₙ-phloridzin) as antiproliferative agent and as glucose regulative agent; therefore useful for treating or preventing a cancer, a metabolism disorder, diabetes or the obesity;
- [6)-O-α-D-Glcp-(1→]ₙ-6-O⁸-β-D-Glcp-(1→-gossypetin (also named [6)-O-α-D-Glcp-(1→]ₙ-gossypin) as an anti-inflammatory or analgesic agent; therefore useful for treating or preventing an inflammatory disease, an immune disorder or pain;
- [6)-O-α-D-Glcp-(1→4]ₙ-6-O^{4'}-β-D-Glcp-(1→-quercetin (also named [6)-O-α-D-Glcp-(1→]ₙ-spiraeoside) as antiproliferative or antioxidant agent; therefore useful for treating or preventing a cancer, or a brain disease;
- [6)-O-α-D-Glcp-(1→]ₙ-sennoside A or [6)-O-α-D-Glcp-(1→]ₙ-sennoside B as laxative agent or alpha-glucoamylase inhibitor; therefore useful for treating or preventing a metabolism disorder, diabetes or the obesity;
- [6)-O-α-D-Glcp-(1→4]ₙ-6-O³-β-D-Glcp-(1→-3,5-dihydroxy-4'-methoxystilbene (also named [6)-O-α-D-Glcp-(1→]ₙ-deoxyrhapontin) as a glucose regulative agent; therefore useful for treating or preventing a metabolism disorder, diabetes or the obesity;
- [6)-O-α-D-Glcp-(1→]ₙ-6-O³-β-D-Glcp-(1→-resveratrol (also named [6)-O-α-D-Glcp-(1→]ₙ-piceid) as antioxidant agent or ischemia/reperfusion injury protective agent; therefore useful for treating or preventing a cardiovascular disease, or a brain disease;
- [6)-O-α-D-Glcp-(1→]ₙ-6-O³-β-D-Glcp-(1→-4'-methoxy-3,3',5-stilbenetriol (also named [6)-O-α-D-Glcp-(1→]ₙ-rhapontin) as lipid peroxidation inhibitor, anti-proliferative, antithrombotic or antiallergic agent; therefore useful for treating or preventing a metabolism disorder, diabetes, the obesity, a cardiovascular disease, a cancer, an allergy, or an inflammatory disease;
- [6)-O-α-D-Glcp-(1→]ₙ-6-O³-β-D-Glcp-(1→-maackiain (also named [6)-O-α-D-Glcp-(1→]ₙ-trifolirhizin) as antiproliferative agent; therefore useful for treating or preventing a cancer; and,
- [6)-O-α-D-Glcp-(1→]ₙ-6-O⁶-β-D-Glcp-(1→-esculetin (also named [6)-O-α-D-Glcp-(1→]ₙ-esculin) as carcinogenesis preventive agent; therefore useful for treating or preventing a cancer.

Such a composition can comprise pharmaceutically acceptable carrier, stabilizers or excipients.

### Use of phenolic compounds as key intermediates for the development of other derivatives

[6)-O-α-D-Glcp-(1→]ₙ-6-O-β-D-Glcp-(1→-phenolic derivative of the present invention can be directly used as active ingredients as cosmetics or as active substances alone or in combination with other products, including other active molecules with synergistic or complementary activities, or with stabilizers or excipients. These phenolic compounds derivatives can also be used as starting materials for additional chemical, physical or enzymatic modification(s) in order to produce second generation of derivatives. The other hydroxyl groups can for example be used in a chemical reaction to create ester bonds, acyl bonds, ether bonds, sulphate or phosphate bonds. Such modifications can improve already existing properties of the [6)-O-α-D-Glcp-(1→]ₙ-6-O-β-D-Glcp-(1→-phenolic derivative of the present invention, or provide new properties for specific applications (higher therapeutic efficiency, lower cytotoxicity, higher stability after release of the glycone part by micro-organisms, ...).

### Formulation of said derivatives for cosmetic or therapeutic applications

The compositions of the present invention may be administered orally, parenterally, by inhalation spray, topically, rectally, nasally, buccally, vaginally or via an implanted reservoir. The term "parenteral" as used herein includes subcutaneous, intravenous, intramuscular, intra-articular, intra-synovial, intrasternal, intrathecal, intrahepatic, intralesional and intracranial injection or infusion techniques. Preferably, the composition of the present invention are administered orally, by inhalation spray, topically, rectally, nasally, buccally, or vaginally. In a preferred embodiment, the pharmaceutical or cosmetic composition is administered topically.

New types of cosmetic products are constantly being developed, and new raw materials are adding to the cosmetic chemist's selection of personal care ingredients. The [6)-O-α-D-Glcp-(1→]ₙ-6-O-β-D-Glcp-(1→-phenolic derivative described in the present invention can easily be incorporated in a large panel of cosmetic products. Such preparations are well known from the man of the art : it can be creams, sticks, shampoo, shower gels, lotions, soaps, emulsions, gels. These formulations can include other ingredients such as but not limited to : deionized water, magnesium, aluminium silicate, xanthan gum, nylon-12, sodium PCA, propylene glycol, red iron oxides, talc, yellow iron oxides, black iron oxides, titanium dioxide, glyceryl stearate, stearic acid, DEA-cetyl phosphate, methylparaben, butylparaben, ethylparaben, propylparaben, isotearyl neopentanoate, isopropyl palmitate, ethylene/propylene/styrene, copolymers, butylene/ethylene/styrene copolymer, isopropyl palmitate, phenoxyethanol tocopheryl acetate, glycerin, triethanolamine, stearic acid, propylene glycol stearate, mineral oil, buthylene/ethylene/styrene copolymer, diazolidinyl urea, hydrogenated polyisobutene, octyl palmitate, tridecyl neopentanoate, isostearyl isotearate, isopropylparaben, isobutyparaben, octyldodecyl neopentanoate, tocopheryl acetate, fragrance, octyl methoxycinnamate, benzophenone, octyl salicylate, isopropyl isostearate, propylene glycol isoceteth-3 acetate, or any combinations thereof.

For their use in therapeutic applications, [6)-O-α-D-Glcp-(1→]ₙ-6-O-β-D-Glcp-(1→-phenolic derivative of the present invention can be incorporated in different galenic preparations such as pills, tablets, syrups, creams, lotions, gels using for example packing, standardisation, blending / homogenisation, sterile and nonsterile micronization, granulation / compacting, sieving or any combination thereof. Preparations of said [6)-O-α-D-Glcp-(1→]ₙ-6-O-β-D-Glcp-(1→-phenolic derivative can include some excipients of the following non exhaustive list : talc, lactose, magnesium stearate, glycerol monostearate, colloidal silicon dioxide, precipitated silicon dioxide, crosslinked polyvinyl pyrrolidone, dibasic calcium phosphate dihydrate, micro crystalline cellulose, corn starch, povidone, sodium carboxy-methyl cellulose, polysorbate 80, lactic acid, carbomer, cethyl alcohol, isopropyl myristate, isopropyl palmitate, glucose, dextrose, triethanolamine, glycerine, fructose, sucrose, polymers, nanostructures.

The compositions of this invention may be orally administered in any orally acceptable dosage form including, but not limited to, capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include lactose and dried cornstarch. When aqueous suspensions are required for oral use, the active ingredient is combined with emulsifying and suspending agents. If desired, certain sweetening, flavoring or coloring agents may also be added.

Alternatively, the compositions of this invention may be administered in the form of suppositories for rectal administration. These can be prepared by mixing the agent with a suitable non-irritating excipient that is solid at room temperature but liquid at rectal temperature and therefore will melt in the rectum to release the drug. Such materials include cocoa butter, beeswax and polyethylene glycols.

The compositions of this invention may also be administered topically, especially when the target of treatment includes areas or organs readily accessible by topical application, including diseases of the eye, the skin, or the lower intestinal tract. Suitable topical formulations are readily prepared for each of these areas or organs.

For topical applications, the compositions may be formulated in a suitable ointment containing the active component suspended or dissolved in one or more carriers. Carriers for topical administration of the compounds of this invention include, but are not limited to, mineral oil, liquid petrolatum, white petrolatum, propylene glycol, polyoxyethylene, polyoxypropylene compound, emulsifying wax and water. Alternatively, the compositions can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water.

For ophthalmic use, the compositions may be formulated as micronized suspensions in isotonic, pH adjusted sterile saline, or, preferably, as solutions in isotonic, pH adjusted sterile saline, either with or without a preservative such as benzylalkonium chloride. Alternatively, for ophthalmic uses, the compositions may be formulated in an ointment such as petrolatum.

The compositions of this invention may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents. Sterile injectable forms of the compositions of this invention may be aqueous or an oleaginous suspension. These suspensions may be formulated according to techniques known in the art using suitable dispersing or wetting agents and suspending agents. The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

### ADVANTAGES OF THE PRESENT INVENTION

The advantages of the compounds of the present invention over pre-existing compounds appears clearly from the previous descriptions and embodiments. A non exhaustive list of others advantages of the present invention are described below.

The present invention describes new original [6)-O-α-D-Glcp-(1→]ₙ-6-O-β-D-Glcp-(1→-phenolic derivative of daidzein, genistein, formononetin, sissotrin, apigenin, isovitexin, naringenin, luteolin, kaempferol, eriodictyol, isookanin, 2',4',6'-trihydroxydihydrochalcone, phloretin, okanin, gossypin, maritimetin, quercetin, salicylaldehyde, sennidine A or B, 3,5-dihydroxy-4'-methoxystilbene, resveratrol, and 4'-methoxy-3,3',5-stilbenetriol, and derivatives thereof.

These [6)-O-α-D-Glcp-(1→]ₙ-6-O-β-D-Glcp-(1→-phenolic derivative of high interest in the fields of cosmetic, nutrition and therapy as they show improved water solubility. Indeed, an increase by at least 50% and up to 5000% of the soblubility has been observed in comparison with the corresponding O-β-D-Glcp-(1→-phenolic derivative in the same physiological conditions.

These [6)-O-α-D-Glcp-(1→]ₙ-6-O-β-D-Glcp-(1→-phenolic derivative have an increased bioavailability. These [6)-O-α-D-Glcp-(1→]ₙ-6-O-β-D-Glcp-(1→-phenolic derivative can be "in situ released" through their hydrolysis into the initial phenolic or glycosyl-phenolic structure by enzymes of human cells and/or of human commensal micro-organisms, giving them a "pro-drug" status of high interest for both cosmetic, nutrition and therapy applications.

### EXAMPLES

Any other embodiments and advantages of the present invention will appear from the following examples, that are illustrative of embodiments of the invention and are not meant to limit the scope of the invention as encompassed by the claims.

### EXAMPLE 1 : Obtention of (6)-O-α-D-Glcp-(1→]ₙ-6-O^{2'}-β-D-Glcp-(1→-phloretin derivatives

Reaction media were prepared as described in EP2007/055815 with phloridzin ([Extrasynthese; 69730 Genay, France], reference [1046]).

After 21 hours of incubation, a sample of each reaction medium was diluted 50 times with a solution containing methanol and water in the proportions of 40/60 and then analysed by HPLC (method N#1).

The results are reported in the following table.

| Glucosyl acceptor | Retention time, minutes | m/z [M-H] | Identification (theroretical molecular weight) |
|---|---|---|---|
| phloridzin (chromatogram at 285 nm in Figure 12) | 9,63 | 435.4 | O^{2'}-β-D-Glcp-(1→-phloretin or phloridzin (436.4) |
| | 8,20 | 597.6 | [6)-O-α-D-Glcp-(1→]₁-6-O^{2'}-β-D-Glcp-(1→-phloretin or [6)-O-α-D-Glcp-(1→]₁-phloridzin (598.4) |
| | 7,72 | 759.7 | [6)-O-α-D-Glcp-(1→]₂-6-O^{2'}-β-D-Glcp-(1→-phloretin or [6)-O-α-D-Glcp-(1→]₂-phloridzin (760.4) |
| | 7,35 | 921.8 | [6)-O-α-D-Glcp-(1→]₂-6-O^{2'}-β-D-Glcp-(1→-phloretin or [6)-O-α-D-Glcp-(1→]₂-phloridzin (922.4) |

### EXAMPLE 2 : Obtention of [6)-O-α-D-Glcp-(1→]ₙ-6-O³-β-D-Glcp-(1→-4'-methoxy-3,3',5-stilbenetriol and [6)-O-α-D-Glcp-(1→]ₙ-6-O³-β-D-Glcp-(1→-3,5-dihydroxy-4'-methoxystilbene derivatives

Reaction media were prepared as described in EP2007/055815 with a mixture of rhapontin (4'-methoxy-3,3',5-stilbenetriol-3-O-β-D-glucopyranoside) and deoxyrhapontin (3,5-dihydroxy-4'-methoxystilbene-3-O-β-D-glucopyranoside) ([Extrasynthese], reference [6591]).

After 21 hours of incubation, a sample of each reaction medium was diluted n times with a solution containing methanol and water in the proportions of 40/60 and then analysed by HPLC (method N#2).

The results are reported in the following table.

| Glucosyl acceptor | Retention time, minutes | m/z [M-H] | Identification (theroretical molecular weight) |
|---|---|---|---|
| Rhapontin and deoxyrhapontin (chromatogram at 320 nm in Figure 13) | 7.42 | 403.0 | 3,5-dihydroxy-4'-methoxystilbene-3-O-β-D-glucopyranoside or deoxyrhapontin (404.4) |
| | 6.87 | 565.0 | [6)-O-α-D-Glcp-(1→]₁-deoxyrhapontin (566.4) |
| | 6.47 | 727.0 | [6)-O-α-D-Glcp-(1→]₂-deoxyrhapontin (728.4) |
| | 5.18 | 419.0 | 4'-methoxy-3,3',5-stiibenetriol-3-O-β-D-glucopyranoside or rhapontin (420.4) |
| | 4.58 | 581.0 | [6)-O-α-D-Glcp-(1→]₁-rhapontin (582.4) |

### EXAMPLE 3 : (6)-O-α-D-Glcp-(1→]ₙ-6-O⁷-β-D-Glcp-(1→-naringenin derivatives

Reaction media were prepared as described in EP2007/055815 with naringenin-7-O-β-D-glucopyranoside or prunin ([Extrasynthese], reference [1090]).

After 21 hours of incubation, a sample of each reaction medium was diluted n times with a solution containing methanol and water in the proportions of 40/60 and then analysed by HPLC (method N#1).

The results are reported in the following table.

| Glucosyl acceptor | Retention time, minutes | m/z [M+H] | Identification (theroretical molecular weight) |
|---|---|---|---|
| naringenin-7-O-β-D-glucopyranoside (prunin) (chromatogram at 284 nm in Figure 14) | 9,17 | 435.4 | O⁷-β-O-Glcp-(1→-naringenin or prunin (434.4) |
| | 8,03 | 597.4 | [6)-O-α-D-Glcp-(1→]₁-6-O⁷-β-D-Glcp-(1→4-naringenin or [6)-O-α-D-Glcp-(1→]₁-prunin (596.4) |
| | 7,52 | 759.4 | [6)-O-α-D-Glcp-(1→]₂-6-O⁷-β-D-Glcp-(1→-naringenin or [6)-O-α-D-Glcp-(1→]₂-prunin (758.4) |
| | 7,19 | 921.6 | [6)-O-α-D-Glcp-(1→]₃-6-O⁷-β-D-Glcp-(1→-naringenin or [6)-O-α-D-Glcp-(1→]₃-prunin (920.4) |

### EXAMPLE 4 : Obtention of [6)-O-α-D-Glcp-(1→]ₙ-6-O³-β-D-Glcp-(1→-resveratrol derivatives

Reaction media were prepared as described in EP2007/055815 with resveratrol-3-O-glucoside ([Extrasynthese], reference [4992]).

After 21 hours of incubation, a sample of each reaction medium was diluted n times with a solution containing methanol and water in the proportions of 40/60 and then analysed by HPLC (method N#1).

The results are reported in the following table.

| Glucosyl acceptor | Retention time, minutes | m/z [M-H] | Identification (theroretical molecular weight) |
|---|---|---|---|
| Resveratrol-3-O-β-D-glucopyranoside or piceid (chromatogram at 318 nm in Figure 15) | 6.80 | 389.2 | O³-β-D-Glcp-(1→)-resveratroi or piceid (390.4) |
| | 6.63 | 551.2 | [6)-O-α-D-Glcp-(1→]₁-6-O³-β-D-Glcp-(1→-resveratrol or [6)-O-α-D-Glcp-(1→]₁-piceid (552.4) |
| | 6.20 | 551.2 | [6)-O-α-D-Glcp-(1→]₁-6-O³-β-D-Glcp-(1→-resveratrol or [6)-O-α-D-Glcp-(1→]₁-piceid (552.4) |
| | 5.80 | 713.2 | [6)-O-α-D-Glcp-(1→]₂-6-O³-β-D-Glcp-(1→-resveratrol or [6)-O-α-D-Glcp-(1→]₁-piceid (714.4) |

### EXAMPLE 5 : [6)-O-α-D-Glcp-(1→]n-6-O⁶-β-D-Glcp-(1→-esculetin derivatives

Reaction media were prepared as described in EP2007/055815 with esculin ([Sigma-Aldrich; 38297 Saint Quentin Fallavier, France], reference [E8250]).

After 21 hours of incubation, a sample of each reaction medium was diluted n times with a solution containing methanol and water in the proportions of 40/60 and then analysed by HPLC (method N#3).

The results are reported in the following table.

| Glucosyl acceptor | Retention time, minutes | m/z [M-H] | Identification (theroretical molecular weight) |
|---|---|---|---|
| Esculin (chromatogram at 335 nm in Figure 16) | 18.15 | 176.9 | Esculetin or 6,7-dihydroxy-coumarin (178.1) |
| | 13.52 | 339.0 | O⁶-β-D-Glcp-(1→-esculetin or esculin (340.3) |
| | 11.27 | 501.0 | [6)-O-α-D-Glcp-(1→]₁-6-O⁶-β-D-Glcp-(1→-escuietin or [6)-O-α-D-Glcp-(1→]₁-esculin (502.3) |
| | 10.57 | 663.1 | [6)-O-α-D-Glcp-(1→]2-6-O⁶-β-D-Glcp-(1→-esculetin or [6)-O-α-D-Glcp-(1→]₂-esculin (664.3) |
| | 9.22 | 1149.5 | [6)-O-α-D-Glcp-(1→]₅-6-O⁶-β-D-Glcp-(1→-esculetin or [6)-0-α-D-Glcp-(1→]₅-esculin (1050.3) |

### EXAMPLE 5 : Release of rhapontin from [6)-O-α-D-Glcp-(1→]ₙ-6-O-β-D-Glcp-(1→4'-methoxy-3,3',5-stilbenetriol and [6)-O-α-D-Glcp-[1→]ₙ-6-O³-β-D-Glcp-(1→-3,5-dihydroxy-4'-methoxystilbene derivatives by an α-glucosidase preparation

[6)-O-α-D-Glcp-(1→]ₙ-6-O-β-D-Glcp-(1→-4'-methoxy-3,3',5-stilbenetriol and [6)-O-α-D-Glcp-(1→]ₙ-6-O³-β-D-Glcp-(1→-3,5-dihydroxy-4'-methoxystilbene derivatives were incubated in the presence of an α-glucosidase enzyme in the following conditions:
   [6)-O-α-D-Glcp-(1→]ₙ-6-O-β-D-Glcp-(1→-4'-methoxy-3,3',5-stilbenetriol and [6)-O-α-D-Glcp-(1→]ₙ-6-O³-β-D-Glcp-(1→-3,5-dihydroxy-4'-methoxystilbene derivatives obtained as described in example 2 : 0.20 ml; α-glucosidase (from *Saccharomyces cerevisiae ;* FLUKA 70797; lot 0641337/1; activity: 5.8 U/mg): 10 mg in 10 ml of PIPES buffer 0.025 M, pH 6.3; no enzyme in the control reaction medium;
   Temperature: 30°C; Moderate agitation.

The reaction media were analysed by HPLC after a 2-fold dilution of an aliquote with methanol.

After 3 hours of incubation, the [6)-O-α-D-Glcp-(1→]ₙ-6-O-β-D-Glcp-(1→-4'-methoxy-3,3',5-stilbenetriol and [6)-O-α-D-Glcp-(1→4]ₙ-6-O³-β-D-Glcp-(1→-3,5-dihydroxy-4'-methoxystilbene derivatives remained unchanged in the medium which did not contain the α-glucosidase enzyme whereas the [6)-O-α-D-Glcp-(1→4]ₙ-6-O-β-D-Glcp-(1→-4'-methoxy-3,3',5-stilbenetriol and [6)-O-α-D-Glcp-(1→4]ₙ-6-O³-β-D-Glcp-(1→-3,5-dihydroxy-4'-methoxystilbene derivatives were totally converted into rhapontin and deoxyrhapontin in the presence of the α-glucosidase enzyme.

These results show that an isolated enzyme specific for the hydrolysis of α-glucosidic bonds is able to hydrolyse the [6)-O-α-D-Glcp-(1→]ₙ-6-O-β-D-Glcp-(1→-4'-methoxy-3,3',5-stilbenetriol and [6)-O-α-D-Glcp-(1→]ₙ-6-O³-β-D-Glcp-(1→-3,5-dihydroxy-4'-methoxystilbene derivatives : this indicates that the [6)-O-α-D-Glcp-(1→]ₙ-6-O-β-D-Glcp-(1→-4'-methoxy-3,3',5-stilbenetriol and [6)-O-α-D-Glcp-(1→]ₙ-6-O³-β-D-Glcp-(1→-3,5-dihydroxy-4'-methoxystilbene derivatives contains either rhapontin or deoxyrhapontin and glucose(s) with glucose(s) being linked to an hydroxyl group of rhapontin or deoxyrhapontin through an α-glucosidic bond.

### EXAMPLE 6 : Compared solubility in water of some [6)-O-α-D-Glcp-(1→]₁-6-O-β-D-Glcp-(1→-phenolic derivatives with their corresponding O-β-D-Glcp-(1-phenolic derivatives

Solubility in water of several [6)-O-α-D-Glcp-(1→]₁-6-O-β-D-Glcp-(1→-phenolic derivatives have been evaluated and compared to solubility of their corresponding O-β-D-Glcp-(1→-phenolic derivatives in the same conditions of pH (ranging from 4.0 to 5.0), salinity (no buffer added) and temperature (25°C).

For this purpose, these [6)-O-α-D-Glcp-(1→]ₙ-6-O-β-D-Glcp-(1→-phenolic derivatives have been prepared as described in example 1, and concentrated as described in EP2007/055815. The resulting concentrated syrup obtained after purification of these [6)-O-α-D-Glcp-(1→]₁-6-O-β-D-Glcp-(1→-phenolic derivatives has been diluted and analyzed by HPLC to estimate the content of soluble [6)-O-α-D-Glcp-(1→]₁-6-O-β-D-Glcp-(1→-phenolic derivatives.

In parallel, an excess amount of each corresponding β-D-Glcp-(1→-phenolic derivatives has been diluted in water to enable the preparation of water saturated solutions of these compounds. After high speed centrifugation to remove the excess of solid material, the supernatant of these saturated solutions has been analyzed by HPLC to estimate the content of soluble O-β-D-Glcp-(1→-phenolic derivatives.

Results are mentioned in the table below.

Nota : One should notice that solubility of the [6)-O-α-D-Glcp-(1→]₁-6-O-β-D-Glcp-(1→-phenolic derivatives is considered as a minimal solubility as concentration of the initial syrups have not been tested.

| | | |
|---|---|---|
| A= solubility in water of [6)-O-α-D-Glcp-(1→]₁-6-O-β-D-Glcp-(1→-phenolic compound (g/L) [mM] | B= solubility in water of O-β-D-Glcp-(1→-phenolic compound (g/L) [mM] | % of solubility increase =(A-B)/Bx100 |
| [6)-O-α-D-Glcp-(1→]₁-6-O^{2'}-β-D-Glcp-(1→-phloretin higher than 53.3 g/L [89.1 mM] | Phloridzin 1,1 g/L [2.5 mM] | In g/L Higher than 4750 % In mM Higher than 3450 % |
| [6)-O-α-D-Glcp-(1→]₁-6-O³-β-D-Glcp-(1→-4'-methoxy-3,3',5-stilbenetriol higher than 16.6 g/L [28.5 mM] | Rhapontin 0,3 g/L [0.7 mM] | In g/L Higher than 5400 % In mM Higher than 3950 % |
| [6)-O-α-D-Glcp-(1→]₁-6-O³-β-D-Glcp-(1→-3,5-dihydroxy-4'-methoxystilbene higher than 8.5 g/L [15.0 mM] | Deoxyrhapontin 0.3 g/L [0.7 mM] | In g/L Higher than 2700 % In mM Higher than 2000 % |
| [6)-O-α-D-Glcp-(1→]₁-6-O³-β-D-Glcp-(1→-resveratrol higher than 65.0 g/L [117.6 mM] | O-β-D-Glcp-(1→-resveratrol Resveratrol 1.2 g/L [3.1 mM] | In g/L Higher than 5300 % In mM Higher than 3650 % |
| [6)-O-α-D-Glcp-(1→]₁-6-O⁶-β-D-Glcp-(1→-esculetin higher than 12.5 g/L [24.9 mM] | Esculin 1.3 g/L [3.8 mM] | In g/L Higher than 850 % In mM Higher than 550 % |

All these [6)-O-α-D-Glcp-(1→]ₙ-6-O-β-D-Glcp-(1→-phenolic derivatives show at least 50% of increase fo their solubility in water as compared to their corresponding O-β-D-Glcp-(1→-phenolic derivatives.

## Claims

1. Water soluble [6)-O-α-D-Glcp-(1→]ₙ-6-O-β-D-Clcp-(1→-phenolic derivative having the following formula (I) : 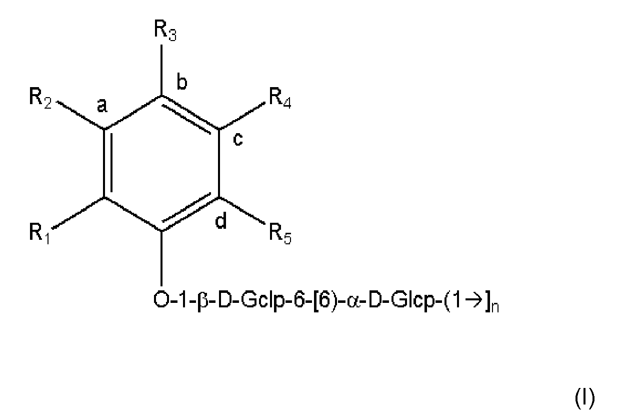 wherein
n is equal or greater than 1; more preferably n=1, 2, 3 or 4 and much more preferably n=1,
R1 is selected from the group consisting of H, OH and OCH₃, preferably H or OH,
R2 is selected from the group consisting of H, OH, OCH₃, and 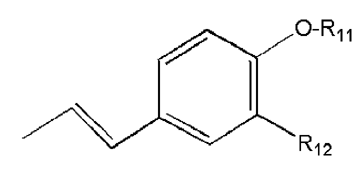
wherein R11 is H or CH₃ and R12 is H or OH,
and,
R3 is selected from the group consisting of H, OH, COCH₃, 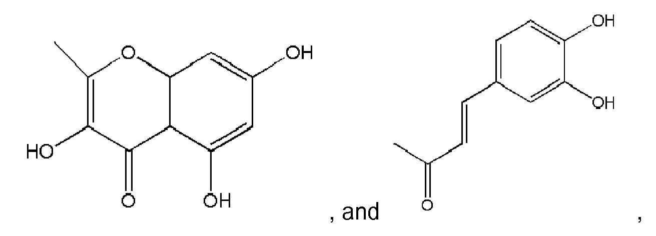
or,
R2 and R3 can together form an heteroring, when taken together with the atoms to which they are attached, said heterocyle being selected among the group consisting of: 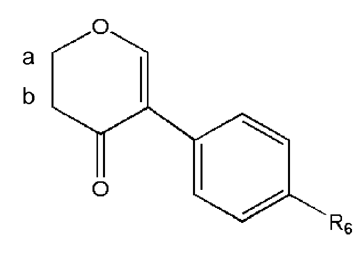 wherein R6 is H, OH or OCH₃, 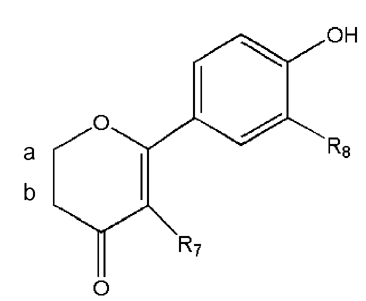 wherein R7 and R8 are selected from the group consisting of H, OH and OCH₃, preferably are H or OH, 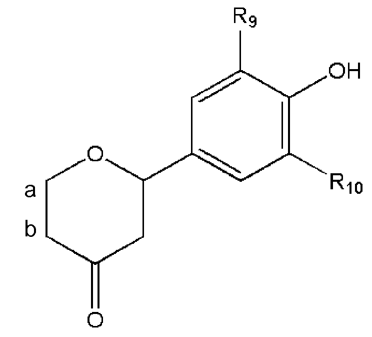 wherein R9 and R10 are selected from the group consisting of H, OH and OCH₃, preferably are H or OH, 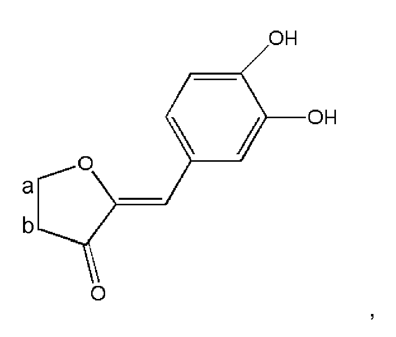 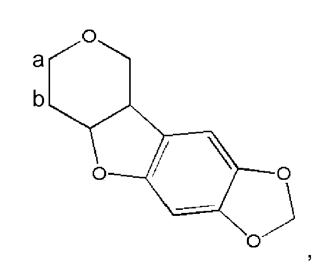 and 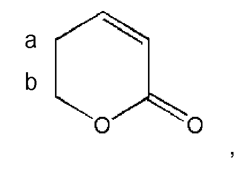
R4 is selected from the group consisting of H, OH and OCH₃, preferably is H or OH,
and
R5 is selected from the group consisting of H, OH, 1-C-glucoside, COH, 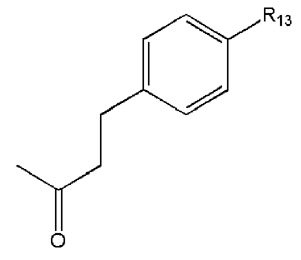
wherein R13 is selected from the group consisting of H, OH and OCH₃, preferably is H or OH,
or,
R4 and R5 can together form a ring, when taken with the atoms to which they are attached, said heterocyle being selected among the group consisting of: 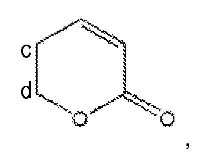 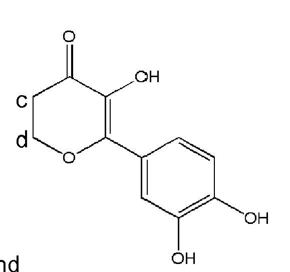 and 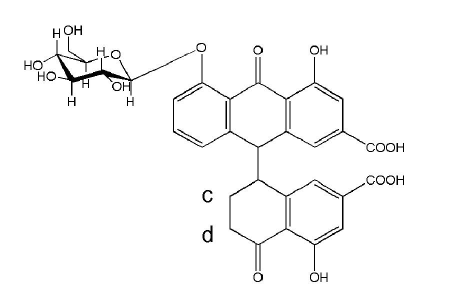 with the provisio that when R1, R2, R4, R5 are H then R3 is not OH,
or any salt thereof, in particular any pharmaceutically acceptable salt thereof.

2. The [6)-O-α-D-Glcp-(1→]ₙ-6-O-β-D-Glcp-(1→-phenolic derivative according to claim 1 wherein R1, R2, R3, R4, R5 are selected among the following combinations:
a) R1 and R5 are H; R4 is H or OH; R2 and R3 together form, when taken together with the atoms to which they are attached, form a 6-membered heteroring, substituted by a phenol or phenoxy, of formula : 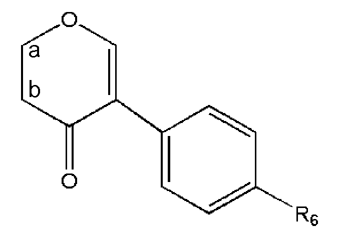 wherein R6 is OH or OCH₃; or
b) R1 is H, R4 is H or OH, R5 is H or 1-C-glucopyranoside, preferably at least one and only one among R4 and R5 being H, R2 and R3 together form when taken together with the atoms to which they are attached, a 6-membered heteroring, substituted by a phenol, of formula : 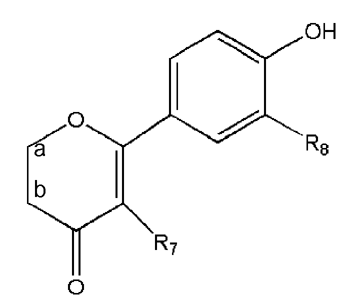 wherein R7 and R8 are H or OH, preferably R7 and R8 being not both OH, and more preferably when R7 or R8 represents OH, then R4 and R5 represent OH and H, respectively; or
c) R5 is H, R1 and R4 are H or OH, preferably at least one and only one among R1 and R4 being H, R2 and R3 together form, when taken together with the atoms to which they are attached, a 6-membered heteroring, substituted by a phenol, of formula : 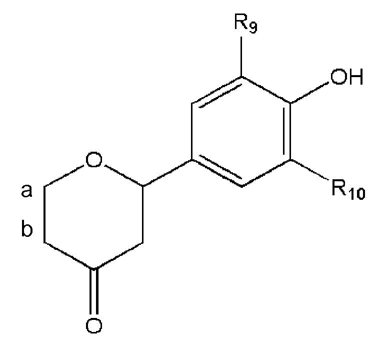 wherein R9 and R10 are H or OH, preferably R9 and R10 being not both OH; or
d) R1, R3 and R5 are H, R4 is OH,and R2 is 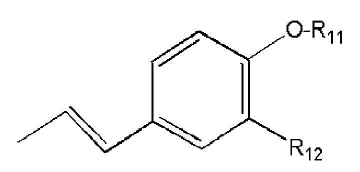 wherein R11 is H or CH3 and R12 is H or OH; or
e) R1 and R3 are H, R2 and R4 are OH, and R5 is 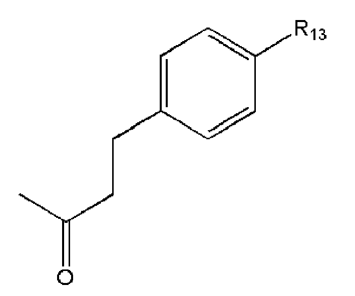 wherein R13 is H or OH, or
f) R1 and R2 are H, R5 is OH, R4 is H or OH and R3 is selected from the group consisting of 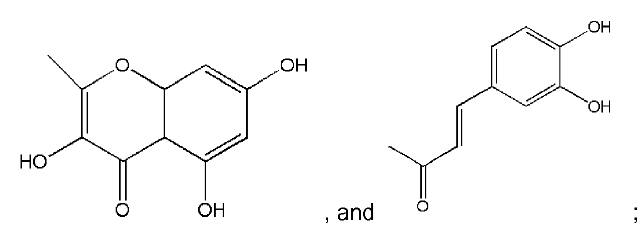 or
g) R1, R2 and R4 are H, R3 and R5 are either respectively COCH₃ and H, or H and COH; or
h) R1 is H or OH, R4 and R5 are H, R2 and R3 together form, when taken together with the atoms to which they are attached, a heteroring selected from the group consisting of: 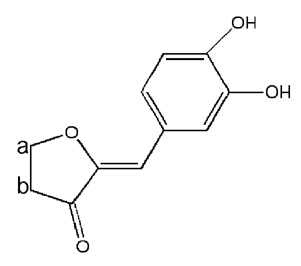 and 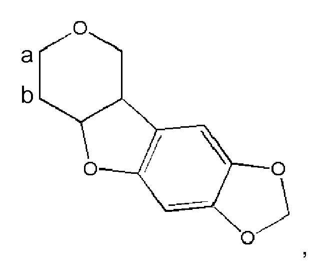 and preferably, when said heteroring is a 6-member ring, then R1 is H, and when said heteroring is a 5-member ring, then R1 is OH; or
i) R5 is OH, R1 and R4 are H, R2 and R3 together form, when taken together with the atoms to which they are attached, a 6-member heteroring of formula: 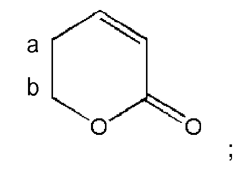 or
j) R1 and R3 are H or OH, R2 is H or OCH₃, R4 and R5 together form, when taken together with the atoms to which they are attached, a 6-member ring of formula selected in the group consisting of: 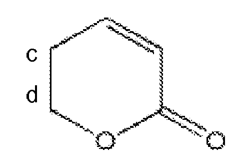 preferably with R1, R2 and R3 being OH, OCH₃ and H, respectively, 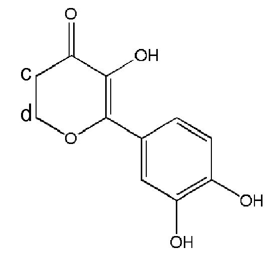 preferably with R1 and R3 being OH, and R2 being H, and 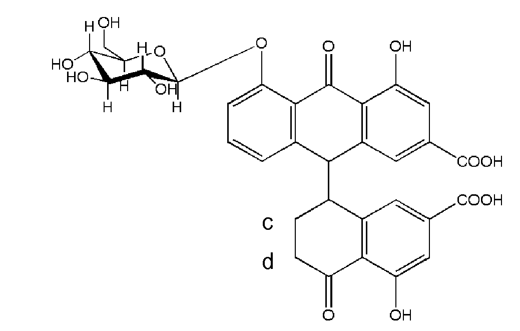 preferably with R1, R2 and R3 being all H.

3. The [6)-O-α-D-Glcp-(1→]ₙ-6-O-β-D-Glcp-(1→-phenolic derivative of claim 1 or 2, wherein the [6)-O-α-D-Glcp-(1→]ₙ-6-O-β-D-Glcp-(1→-phenolic derivative is selected in the group consisting of [6)-O-α-D-Glcp-(1→]ₙ-6-O-β-D-Glcp-(1→-daidzein (also named [6)-O-α-D-Glcp-(1→]ₙ-daidzin), [6)-O-α-D-Glcp-(1→]ₙ-6-O⁷-β-D-Glcp-(1→-genistein (also named [6)-O-α-D-Glcp-(1→]ₙ-genistin), [6)-O-α-D-Glcp-(1→]ₙ-6-O⁷-β-D-Glcp-(1→-formononetin (also named [6)-α-D-Glcp-(1→]ₙ-ononin), [6)-O-α-D-Glcp-(1→]ₙ-6-O⁷-β-D-Glcp-(1→-biochanin A (also named [6)-O-α-D-Glcp-(1→]ₙ-sissotrin), [6)-O-α-D-Glcp-(1→]ₙ-6-O⁷-β-D-Glcp-(1→-apigenin (also named [6)-O-α-D-Glcp-(1→]ₙ-apigetrin), [6)-O-α-D-Glcp-(1→]ₙ-6-O⁷-β-D-Glcp-(1→-isovitexin (also named [6)-O-α-D-Glcp-(1→]ₙ-saponarin), [6)-O-α-D-Glcp-(1→]ₙ-6-O⁷-β-D-(1→-luteolin (also named [6)-O-α-D-Glcp-(1→]ₙ-cynaroside), [6)-O-α-D-Glcp-(1→]ₙ-6-O⁷-β-D-Glcp-(1→-kaempferol (also named [6)-O-α-D-Glcp-(1→]ₙ-populnin), [6)-O-α-D-Glcp-(1→]ₙ-6-O⁷-β-D-Glcp-(1→-naringenin (also named [6)-O-α-D-Glcp-(1→]ₙ-prunin), [6)-O-α-D-Glcp-(1→]ₙ-6-O⁷-β-D-Glcp-(1→-eriodictyol (also named [6)-O-α-D-Glcp-(1→]ₙ-pyracanthoside), [6)-O-α-D-Glcp-(1→]ₙ-6-O⁷-β-D-Glcp-(1→-isookanin (also named [6)-O-α-D-Glcp-(1→]ₙ-flavanomarein), [6)-O-α-D-Glcp-(1→]ₙ-6-O³-β-D-Glcp-(1→-3,5-dihydroxy-4'-methoxystilbene (also named [6)-O-α-D-Glcp-(1→]ₙ-deoxyrhapontin), [6)-O-α-D-Glcp-(1→]ₙ-6-O³-β-D-Glcp-(1→-resveratrol (also named [6)-O-α-D-Glcp-(1→]ₙ-piceid), [6)-O-α-D-Glcp-(1→]ₙ-6-O³-β-D-Glcp-(1→-4'-methoxy-3,3',5-stilbenetriol (also named [6)-O-α-D-Glcp-(1→]ₙ-rhapontin), [6)-O-α-D-Glcp-(1→]ₙ-6-O^{2'}-β-D-Glcp-(1→-2',4',6'-trihydroxydihydrochalcone (also named [6)-O-α-D-Glcp-(1→]ₙ-2',4',6'-trihydroxydihydrochalcone-2'-O-glucoside or [6)-O-α-D-Glcp-(1→]ₙ-4-deoxyphloridzin), [6)-O-α-D-Glcp-(1→]ₙ-6-O^{2'}-β-D-Glcp-(1→-phloretin (also named [6)-O-α-D-Glcp-(1→]ₙ-phloridzin), [6)-O-α-D-Glcp-(1→]ₙ-6-O^{2'}-β-D-Glcp-(1→-okanin (also named [6)-O-α-D-Glcp-(1→]ₙ-marein), [6)-O-α-D-Glcp-(1→]ₙ-6-O^{4'}-β-D-Glcp-(1→-quercetin (also named [6)-O-α-D-Glcp-(1→]ₙ-spiraeoside), [6)-O-α-D-Glcp-(1→]ₙ-6-O⁴-β-D-Glcp-(1→-4-hydroxyacetophenone (also named [6)-O-α-D-Glcp-(1→]ₙ-picein), [6)-O-α-D-Glcp-(1→]ₙ-6-O²-β-O-Glcp-(1→-salicylaldehyde (also named [6)-O-α-D-Glcp-(1→]ₙ-helicin), [6)-O-α-D-Glcp-(1→]ₙ-6-O⁶-β-D-Glcp-(1→-maritimetin (also named [6)-O-α-D-Glcp-(1→]ₙ-maritimein), [6)-O-α-D-Glcp-(1→]ₙ-6-O³-β-O-Glcp-(1→-maackiain (also named [6)-O-α-D-Glcp-(1→]ₙ-trifolirhizin), [6)-O-α-D-Glcp-(1→]ₙ-6-O⁶-β-D-Glcp-(1→-esculetin (also named [6)-O-α-D-Glcp-(1→]ₙ-esculin), [6)-O-α-D-Glcp-(1→]ₙ-6-O⁸-β-D-Glcp-(1→-fraxetin (also named [6)-O-α-D-Glcp-(1→]ₙ-fraxin), [6)-O-α-D-Glcp-(1→]ₙ-6-O⁸-β-D-Glcp-(1→-gossypetin (also named [6)-O-α-D-Glcp-(1→]ₙ-gossypin), [6)-O-α-D-Glcp-(1→]ₙ-sennoside A and [6)-O-α-D-Glcp-(1→]ₙ-sennoside B.

4. The [6)-O-α-D-Glcp-(1→]ₙ-6-O-β-D-Glcp-(1→-phenolic derivative of anyone of claims 1-3, wherein n=1.

5. The [6)-O-α-D-Glcp-(1→]ₙ-6-O-β-D-Glcp-(1→-phenolic derivative of anyone of claims 1-3, wherein n is greater than 1.

6. The [6)-O-α-D-Glcp-(1→]ₙ-6-O-β-D-Glcp-(1→-phenolic derivative according to claim 1, having a solubility in water which is at least from 50% higher than the solubility of the corresponding O-β-D-Glcp-(1→-phenolic derivative in the same conditions.

7. The [6)-O-α-D-Glcp-(1→]ₙ-6-O-β-D-Glcp-(1→-phenolic derivative according to anyone of claims 1 to 6, wherein said [6)-O-α-D-Glcp-(1→]ₙ-6-O-β-D-Glcp-(1→-phenolic derivative can be cleaved by enzymes to release either the corresponding aglycone, or the corresponding [6)-O-α-D-Glcp-(1→]ₙ₋ₓ-6-O-β-D-Glcp-(1→-phenolic derivative wherein x=1 to n.

8. The [6)-O-α-D-Glcp-(1→]ₙ-6-O-β-D-Glcp-(1→-phenolic derivative according to claim 7 wherein said enzymes are glycosidases.

9. The [6)-O-α-D-Glcp-(1→]ₙ-6-O-β-D-Glcp-(1→-phenolic derivative according to claim 7 wherein said enzymes are α-glucosidases and β-glucosidases.

10. The [6)-O-α-D-Glcp-(1→]ₙ-6-O-β-D-Glcp-(1→-phenolic derivative according to claim 7, wherein said enzyme is issued from human associated micro-organisms, in particular human skin associated micro-organisms.

11. A [6)-O-α-D-Glcp-(1→0]ₙ-6-O-β-D-Glcp-(1→-phenolic derivative according to any one of claims 1-10 as medicament.

12. A nutritional, pharmaceutical, dermatological or cosmetic composition comprising a [6)-O-α-D-Glcp-(1→]ₙ-6-O-β-D-Glcp-(1→-phenolic derivative according any one of claims 1-10.

13. The nutritional, pharmaceutical, dermatological or cosmetic composition according to claim 12 further comprising a glycosidase or a micro-organism expressing said glycosidase activity.

14. The composition according to claim 12 or 13 for cosmetic use.

15. Product containing a [6)-O-α-D-Glcp-(1→]ₙ-6-O-β-D-Glcp-(1→-phenolic derivative according any one of claims 1-10 and a glycosidase or a micro-organism expressing said glycosidase activity as a combined preparation for simultaneous, separate or sequential use.

16. Use of a [6)-O-α-D-Glcp-(1→]ₙ-6-O-β-D-Glcp-(1→-phenolic derivative according any one of claims 1-10 for preparing a pharmaceutical or cosmetic composition to be administered topically, orally, rectally, nasally, or vaginally, wherein enzymes issued from micro-organisms associated to skin, mouth, intestinal tract, upper respiratory system or female genital tract release the corresponding aglycone.

17. Use of a [6)-O-α-D-Glcp-(1→]ₙ-6-O-β-D-Glcp-(1→-phenolic derivative according any one of claims 1-10, a composition of claims 12-14, or a product of claim 15 for preparing a pharmaceutical or cosmetic composition for treating or preventing a cancer, a cardiovascular disease, a bacterial infection, a UV-induced erythema, an allergy, an inflammatory or immune disorder.
